# EUROPÄISCHE PATENTSCHRIFT

(11) **EP 2 654 615 B1**
(45) Veröffentlichungstag und Bekanntmachung des Hinweises auf die Patenterteilung: **22.07.2015**
(21) Anmeldenummer: 10807701.7
(22) Anmeldetag: 23.12.2010
(51) Int. Cl.: A61F 2/00

(54) **(TEIL-) VORRICHTUNG ZUR VERHINDERUNG VON INKONTINENZ MIT EINER FIXIEREINRICHTUNG ZUR ORTSFESTEN IMPLANTATION IN KÖRPERGEWEBE**
(PARTIAL) DEVICE FOR PREVENTING INCONTINENCE HAVING A FASTENING APPARATUS FOR STATIONARY IMPLANTATION IN BODY TISSUE
DISPOSITIF (PARTIEL) POUR EMPÊCHER L'INCONTINENCE AVEC UN APPAREIL DE FIXATION POUR L'IMPLANTATION FIXE DANS DU TISSU CORPOREL

(43) Veröffentlichungstag der Anmeldung: 30.10.2013
(73) Patentinhaber: R&M Consulting And Trading Gmbh&CO. KG, 71638 Ludwigsburg (DE)
(72) Erfinder: VITZTHUM, Thomas, 71638 Ludwigsburg (DE)
(74) Vertreter: Wüstefeld, Regine Marie
(86) Internationale Anmeldenummer: PCT/EP2010/007901
(87) Internationale Veröffentlichungsnummer: WO 2012/083993

(56) Entgegenhaltungen:
- WO-A1-00/44319
- WO-A1-98/56311

## Beschreibung

Die Erfindung betrifft eine Vorrichtung zur Verhinderung von Inkontinenz, bzw. einen Teil dieser Vorrichtung, der dann mit einem zweiten Teil zusammenwirkt und eine Fixiereinrichtung zur ortsfesten Implantation in Körpergewebe aufweist. Der Teil der Vorrichtung zur Verhinderung von Inkontinenz weist einen röhrenförmigen Körper auf, mit einem ersten länglichen Führungselement in seinem Inneren. An den röhrenförmigen Körper schließt sich ein schlauchartiges Halteelement an, das in seinem Inneren ein zweites längliches Führungselement aufweist. Dieses endet in axialer Richtung an seiner dem röhrenförmigen Körper entgegengesetzten Seite in einen Abschluß. Das schlauchartige Halteelement ist als ein reversibel dehn- und stauchbares Metallgitter ausgebildet, das einen polymeren Überzug aufweist, mit dem zusammen es, einerseits an dem Abschluß und andererseits in dem Bereich des Übergangs zu dem röhrenförmigen Körper luft- und flüssigkeitsdicht gehalten ist.

Grundsätzlich bezeichnet der Begriff Inkontinenz das Unvermögen, etwas zurückzuhalten. Im Sinne der vorliegenden Erfindung kann dies z. B. Harninkontinenz sein. Ein solcher unwillkürlicher Urinverlust ist eine durchaus häufig auftretende Erkrankung, die verschiedene Ursachen haben kann. Die allgemeine Bezeichnung Harninkontinenz ist daher nur der Oberbegriff für ganz unterschiedliche Krankheitsformen, die sich durch ihre Ursachen unterscheiden und definieren lassen. Neben der Streß- und Belastungsinkontinenz sind die motorische Drang-Inkontinenz und die sensorische Drang-Inkontinenz bekannt. Von diesen wiederum sind die obstruktive Überlauf-Inkontinenz und die funktionelle Überlauf-Inkontinenz, die supraspinale und die spinale Reflex-Inkontinenz oder die extraurethrale Inkontinenz als weitere Ursachen der Inkontinenz zu unterscheiden.

In bezug auf die Belastungsinkontinenz ist von der Firma Uromedica eine Vorrichtung zur Kontrolle der Blasenfunktion bekanntgeworden, die grundsätzlich aus zwei kleinen, implantierbaren Ballons besteht. Diese werden mit einem kurzen operativen Eingriff unter die Haut, neben der Blase implantiert. Die Expansion der Ballons soll vor einem unwillkürlichen Harnabgang schützen, indem so die Harnblase komprimiert wird. Das natürliche Wasserlassen wird auf diese Weise nicht unterbunden, da die Ballongröße so eingestellt wird, daß für das Wasserlassen ein normaler Blasendruck ausreichend ist, um ein Entleeren der Blase zu bewirken. Die Füllmenge der beiden Ballons soll auch nach der Implantation noch durch einen Arzt verändert werden können.

Eine Vorrichtung dieser Art ist durch die WO-A-98/56311 bekanntgeworden. Die WO-A-98/56311 beschreibt eine expandierbare Vorrichtung zur Verhinderung von Harninkontinenz, bei welcher eine röhrenförmige Führung oder Leitung mit einem in bezug auf seinen Umfang veränderbaren, und damit justierbaren Ballon verbunden ist. Dabei durchdringt die röhrenförmige Führung den Ballon axial in der Weise, daß die Führung in den Ballon hinein- und wieder daraus herausragt. Die jeweiligen Verbindungsstellen zwischen Führung und Ballon sind durch Versiegeln, z.B. mittels Silikon, flüssigkeitsdicht verschlossen. Anstelle des Versiegelns mit dem Silikon oder einem vergleichbaren chemischen oder polymeren Klebstoff ist alternativ auch ein Ultraschallschweißen als mögliche Versiegelungstechnik offenbart.

In der röhrenförmigen Führung verläuft in Längsrichtung ein erster Durchlaß, welcher innerhalb des Ballons endet und sich in diesen öffnet. Dieser Durchlaß dient dazu, den Ballon entsprechend den Anforderungen z. B. mit einer Flüssigkeit zu füllen und damit zu expandieren, oder Flüssigkeit herauszuziehen und den Ballon damit zu verkleinern.
Ein weiterer Durchlaß kann vorgesehen sein, der für das Einführen der Vorrichtung im Bereich der Harnröhre eines menschlichen Körpers dient. Entsprechend endet dieser Durchlaß an dem über den Ballon hinaus- und in den Körper hineinreichenden Endbereich der Führung mit einer Öffnung. Seine weitere Öffnung in der Führung weist dieser weitere Durchlaß in dem Bereich zwischen dem Ballon und deren proximalem Ende auf.
Als Material für den Ballon werden zum einen chemische Verbindungen angegeben, die selbst in der Lage sind, mit der röhrenförmigen Führung in den Kontaktbereichen eine Versiegelung zu bewirken. Als solche Verbindungen sind u.a. quervernetztes Silikongel, Polyvinylpyrrolidon und Karayagummi genannt. Neben der schon erläuterten Ausführung der expandierbaren Vorrichtung mit einem ersten Durchlaß, über den der Ballon befüllt oder entleert werden kann, ist in der WO-A-98/56311 auch erläutert, daß die Ballonwand mit einer kernlosen Hohlnadel durchstochen und auf diese Weise befüllt oder entleert werden kann.
Als weiteres Material für den Ballon werden ein biokompatibler nichtrückfederndes Elastomer oder eine entsprechende Polymermischung aus Polyurethan, Polymere wie Polyethylen, Polytetrafluorethylen (PTFE), Polystyrol oder Polyetheretherketon (PEEK) genannt.

Die Wandung des Ballons kann außerdem verstärkende Strukturen aufweisen. Zu diesem Zweck ist der Ballon doppelwandig ausgebildet und die verstärkende Struktur befindet sich dazwischen. Sie kann aus Fasern bestehen, die aus Polyester, Nylon, Polypropylen, Polytetrafluoethylen (wie TEFLON) oder anderen Polymeren bestehen, die einen hohen Härtegrad oder ein hohes Härtemodul aufweisen. Dabei können die Fasern ein Netz bilden, das in eine Trägerstruktur eingewoben ist, die zwischen den Wänden des Ballons angeordnet wird. In einer Ausführungsform können die Fasern weniger elastisch sein, als die Wandung selbst.

Dann weist die gewebte Trägerstruktur eine lose Gewebebindung auf, um so eine Erweiterung oder Verkleinerung der Ballonwandung zu ermöglichen. Andererseits können die Fasern der verstärkenden Struktur auch im wesentlichen nichtelastisch und gewebt sein, wobei die Fasern dann geknotet sind, um so ebenfalls eine Erweiterung oder Verkleinerung der Ballonwandung zuzulassen.

Die verstärkende Struktur hat zusätzlich noch die Aufgabe, in den Ballon eingelassene Teilchen zurückzuhalten, die anstelle einer Flüssigkeit zu seiner Expansion benutzt werden können, damit diese nicht nach außen hin, in den Umgebungsbereich des Ballons entweichen können. Dabei können die in die Trägerstruktur eingewebten Fasern mit einer "Ripstop"-Funktion wasserdicht ausgerüstet sein. Damit sollen beispielsweise auch Tropfen aufgefangen werden können, die sich beim Eindringen in den Ballon mit der Hohlnadel zum Befüllen oder Entleeren bilden.

In bezug auf die Teilchen, welche anstelle oder zusammen mit einer Flüssigkeit zum Befüllen des Ballons verwendet werden können, führt die WO-A-98/56311 verschiedene Beispiele von Teilchen an, die es aufgrund ihrer Größe zunächst erlauben, mittels einer Hohlnadel in den Ballon injiziert zu werden, um erst dort den eigenen Durchmesser zu vergrößern, so daß der Ballon expandiert wird.
Ein Beispiel bilden solche Teilchen, die einen Kern aufweisen, mit einer Vielzahl davon wegweisender Arme. Diese Teilchen passieren zusammengedrückt die Hohlnadeln und expandieren nach dem Eintritt in den Ballon. Andere solcher Teilchen können eine röhrenförmige, langgestreckte Struktur aufweisen, um auf diese Weise die Hohlnadel zu passieren. Die langgestreckte Struktur verhindert dann anschließend ein Zurückfließen der Teilchen durch die Nadel oder die zunächst gebildete Öffnung im Ballon, wenn die Hohlnadel herausgezogen wird. Weitere Ausführungsformen von Teilchen können aus hydrophilem Material bestehen, wie Polyvinylpyrrolidon, Polyethylenglykol, Carboxymethylcellulose oder Hyaluronsäure, das in dem Ballon expandiert.

Durch die PCT/EP2010/003757 ist eine weitere Vorrichtung zur Verhinderung von Inkontinenz bekanntgeworden, bei der anstelle des Ballons aus dem zuvor geschilderten Stand der Technik ein schlauchartiges Haltelement in Form eines reversibel dehn- und stauchbaren Metallgitters eingesetzt wird.
Dieses Metallgitter weist einen polymeren Überzug auf und wird luft- und flüssigkeitsdicht an einem röhrenförmigen Körper in Form eines röhrenförmigen, flexiblen Schlauchs gehalten. Das Metallgitter weist z.B. ein Rautenmuster auf, wobei die Rauten in gestauchtem Zustand als Schlitze ausgebildet sind, die vorzugsweise eine Erstreckung in Längsrichtung des flexiblen Schlauchs eines mit dem genannten schlauchartigen Halteelement verbundenen röhrenförmigen Körpers aufweisen.
Dieser röhrenförmigen Körper dient im wesentlichen dazu, das als schlauchartiges Haltelement bezeichnete Metallgitter an den Ort im Bereich der Harnröhre zu bringen, an dem eine Kontrolle der Blasenfunktion effektiv und sinnvoll erreicht werden kann.
Der röhrenförmige, flexible Schlauch ist als gewebeverstärkter und/oder gewebeumflochtener Schlauch ausgebildet ist, wobei eine einfache oder eine mehrfache Gewebeeinlage vorhanden sein kann. Auch der röhrenförmige, flexible Schlauch kann eine metallische Verstärkung aufweisen.
Der röhrenförmige Körper in Form des röhrenförmigen, flexiblen Schlauchs kann als röhrenförmiger Schrumpfschlauch aus zumindest einem biologisch verträglichen Thermoplasten ausgebildet sein. Das Material ist dann vorzugsweise ausgewählt aus Polyolefinen, Polyvinylchlorid, Polyvinylidenfluorid, Polytetrafluorethylen und/oder Viton. Dieser Schlauch und das schlauchartige Halteelement mit dem Metallgitter gehen dann ineinander über.
Auch der röhrenförmige Schrumpfschlauch kann eine metallische Verstärkung aufweisen. Diese metallische Verstärkung sowie das reversibel dehn- und stauchbare Metallgitter des schlauchartigen Halteelements sind dann vorzugsweise als Sandwich zwischen zwei Kunststofflagen ausgebildet.
Außerdem kann der röhrenförmige Körper gemäß einer seiner Ausführunsgformen eine antimikrobielle Silberbeschichtung oder eine Beschichtung aus diamantähnlichem Kohlenstoff aufweisen.

Im Gegensatz zu der WO-A-98/56311 wird die gemäß dieser PCT-Anmeldung offenbarte Vorrichtung zur Verhinderung von Inkontinenz im Bereich des schlauchartigen Halteelements nicht mit einer Flüssigkeit oder einem sonstigen expandierbaren polymeren Material befüllt, sondern mit Luft von Atmosphärendruck.
Der röhrenförmige Körper weist etwa mittig ein erstes längliches Führungselement in Form einer flexiblen Welle auf, mit Abstandshaltern, die die Welle in dem röhrenförmigen Körper halten. An ihrem distalen Endbereich mündet diese flexible Welle in ein Stellglied, das seinerseits distal endständig einen Eingriff aufweist, um eine manuelle Justierung der Vorrichtung oder eine Justierung mittels eines Motorantriebs zu ermöglichen.

Für den Erfolg einer Vorrichtung zur Verhinderung von Inkontinenz ist es von wesentlicher Bedeutung, daß die einmal beim Implantieren der beiden Teile der Vorrichtung im Bereich der Harnröhre ortsgenau eingesetzten Vorrichtungen nachträglich nicht wandern und so die Zuverlässigkeit der Vorrichtung in Frage stellen.

Gemäß der PCT/EP2010/003757 wird eine Fixierung des röhrenförmigen Körpers und damit der gesamten Vorrichtung am Implantationsort dadurch ermöglicht, daß dieser röhrenförmige Körper an seiner Oberfläche nach außen gerichtete Fixierungselemente in Form von Vorsprüngen, wie Noppen, Haken und/oder schuppenartigen Vorsprüngen aufweist. Diese können in regelmäßigen Abständen z.B. in Reihen oder aber auch in unregelmäßigen Abständen an den röhrenförmigen Körper angeformt sein, wobei sie in der Regel einstückig mit diesem ausgebildet sind.

Eine solche Anordnung von Vorsprüngen der genannten Art am äußeren Umfang des röhrenförmigen Körpers kann zwar die gewünschte Haltefunktion erfüllen und somit dazu beitragen, daß das Implantat auch nach dem Eingriff ortsfest verbleibt, es hat aber Nachteile beim Implantieren selbst. Diese sind darin begründet, daß durch die Vorsprünge, zumindest einen Teil der Ausformungen wie Noppen oder Haken, ein größerer Umfang des röhrenförmigen Körpers bedingt ist, was bei einem minimal invasivem Eingriff möglichst vermieden werden soll.

Andererseits ist auf einem ganz anderen technischen Gebiet, nämlich im Bereich der Wirbelsäulenprothesen, mit der WO-A-00/44319 ein Zwischenwirbeldistanzstück offenbart worden, das expandierbar ist. Solche Zwischenwirbeldistanzstükke werden im wesentlichen benötigt, wenn bei einem Bandscheibenvorfall Teile der Bandscheibe in den Wirbelkanal vortreten, d.h. in den Raum, in dem das Rückmark liegt. Dieser Vorgang verursacht erhebliche Schmerzen. Eine Behandlung wird entweder konservativ durchgeführt oder bei schweren Bandscheibenvorfällen über einen chirurgischen Eingriff. Dabei wird die Bandscheibe ganz oder teilweise entfernt, wobei zwischen die einander benachbarten Wirbel der Wirbelsäule dann ein Distanzstück eingeführt werden muß, in das dann Knochen einwächst, um auf diese Weise die beiden benachbarten Wirbel miteinander zu verbinden. Dies führt an der Stelle des Eingriffs zu einer Wirbelsäulenversteifung. Hierbei war es ein wesentliches Ziel des genannten Standes der Technik, ein Zwischenwirbeldistanzstück bereitzustellen, das einen möglichst geringen chirurgischen Eingriff erfordert und somit selbst einen möglichst geringen Durchmesser beim Implantieren aufweist.
Deshalb hat die WO-A-00/44319 ein Zwischenwirbeldistanzstück vorgeschlagen, das mit einem ersten, kleineren Durchmesser in den Bereich zwischen den beiden betroffenen Wirbeln eingeführt werden und dann zu einem deutlich größeren Durchmesser expandiert werden kann. Dabei kann dieser größere Durchmesser den ersten Durchmesser etwa drei- bis fünfmal oder mehr überragen. Auf diese Weise wird gemäß dem genannten Stand der Technik durch die radiale Expansion des offenbarten Distanzstückes eine optimale Füllung des Zwischenwirbelbereichs erreicht, ohne jedoch einen entsprechend großen chirurgischen Eingriff zu erfordern.
Erreicht wird dies bei dem genannten Stand der Technik dadurch, daß ein axiales Röhrchen vorgesehen ist, das eine Oberfläche, ein proximales und ein distales Ende aufweist. Die Oberfläche des Röhrchens zeigt mehrere Schlitze, die mindesten zwei axial verschobene Fortsätze so definieren, daß sich, wenn das Röhrchen axial zusammengedrückt wird, die Fortsätze aus der Oberfläche heraus erstrecken und eine Geometrie eines expandierten Distanzstücks erzeugen. Diese axial verschobenen Fortsätze umfassen mindestens drei oder vier Fortsätze, die sich entsprechend in mindestens drei oder vier verschiedenen Richtungen von dem Röhrchen heraus erstrecken.
Angewendet auf den Zwischenwirbelbereich bedeutet ein solches axiales Röhrchen, das es zunächst in diesen Zwischenwirbelbereich eingeführt, dann deutlich in seiner Länge gestaucht wird, wobei seitlich in den genannten drei oder vier verschiedenen Richtungen Fortsätze ausfahren, die den Umfang des Röhrchens deutlich vergrößern, somit den Zwischenwirbelbereich ausfüllen, ohne eine entsprechend große, durch den chirurgischen Eingriff bedingte Wunde hervorzurufen.

Ausgehend von diesem Stand der Technik liegt der vorliegenden Erfindung daher die Aufgabe zugrunde, eine Vorrichtung zur Verhinderung von Inkontinenz bereitzustellen, welche die Vorteile der Vorrichtung aufweist, wie sie durch die PCT/EP2010/003757 möglich sind, und gleichzeitig ein ortsfestes Platzieren der beiden Teile der Vorrichtung mit möglichst geringen Nebenwirkungen und minimal invasiv ermöglicht.

Diese Aufgabe wird erfindungsgemäß durch eine Vorrichtung zur Verhinderung von Inkontinenz gelöst, die aus zwei Teilen besteht, und bei welcher zumindest ein Teil der Vorrichtung eine Fixiereinrichtung zur ortsfesten Implantation in Körpergewebe aufweist, mit einem röhrenförmiger Körper und einem ersten länglichen Führungselement in seinem Innern, wobei sich an den röhrenförmigen Körper ein schlauchartiges Halteelement anschließt, das in seinem Innern ein zweites längliches Führungselement aufweist, welches in axialer Richtung an seiner dem röhrenförmigen Körper entgegengesetzten Seite in einen Abschluß mündet, und wobei das schlauchartige Halteelement als ein reversibel dehn- und stauchbares Metallgitter ausgebildet ist, das einen polymeren Überzug aufweist, mit dem zusammen es einerseits an dem Abschluß und andererseits in dem Bereich des

Übergangs zu dem röhrenförmigen Körper luft- und flüssigkeitsdicht gehalten ist. Erfindungsgemäß gekennzeichnet ist dieser zumindest eine Teil der Vorrichtung zur Verhinderung von Inkontinenz dadurch, daß das erste längliche Führungselement die Fixiereinrichtung aufweist, indem das erste längliche Führungselement Bereiche mit axial zueinander verschobenen Vorsprüngen definiert, wobei die Vorsprünge im nicht expandierten Zustand als Schlitze ausgebildet sind, welche nach einem Stauchen des ersten Führungselements in axialer Richtung aus der Oberfläche des Führungselements nach außen treten und unter Bildung der fertigen Vorsprünge aufgestellt sind.

Dadurch wird in besonderem Maße dem Umstand Rechnung getragen, daß sich die Anforderungen an die Beschaffenheit der nach außen weisenden Oberfläche der (Teil-) Vorrichtung zur Verhinderung von Inkontinenz und die Anforderungen an den exakten Sitz nach der Implantation grundsätzlich entgegenstehen können. Während die genannte Oberfläche möglichst glatt sein sollte, um Irritationen des umliegenden Gewebes zu verhindern, ist andererseits eine gute Verankerung in dem Gewebe erforderlich, um ein Verrutschen der Vorrichtung zu vermeiden. Diesem Problem kann grundsätzlich über die hier erfindungsgemäß vorgesehene topologische Oberflächenmodifikation durch die nicht an, sondern in dem röhrenförmigen Körper vorgesehenen Vorsprünge des ersten länglichen Führungselements begegnet werden.
Auf diese Weise kann das erste längliche Führungselement zugleich seine Funktion zur Stabilisierung des röhrenförmigen Körpers erfüllen und die Vorrichtung auf eine sehr gewebeschonende Weise ortsfest halten.

Gemäß einer bevorzugten Ausführungsform verlaufen die an dem ersten länglichen Führungselement ausgebildeten Schlitze im wesentlichen parallel zur Achse des ersten Führungselements. Im gestauchten Zustand ragen die nach außen tretenden Vorsprünge in etwa senkrecht zur Achse nach außen.

Besonders bevorzugt ist es, daß das erste längliche Führungselement, einschließlich der an ihm ausgebildeten Vorsprünge, aus einer Nickel-Titan-Legierung mit einem Form-Gedächtnis-Effekt (Memory-Effekt)gebildet ist. Eine solche Nickel-Titan-Legierung mit einem Form-Gedächtnis-Effekt ist z.B. unter dem Namen Nitinol® im Handel erhältlich.

Das erste längliche Führungselement ist vorzugsweise als Hohlkörper ausgebildet. Wenn dann gemäß einer weiteren Ausbildung des erfindungsgemäßen Teils der Vorrichtung zur Verhinderung von Inkontinenz in das erste längliche Führungselement ein an den Innendurchmesser des Hohlkörpers angepaßter Stab eingeführt ist, der eine vorherbestimmte Krümmung aufweist, die ihrerseits an die Anatomie des Implantationsortes angepaßt ist, kann diese Krümmung auf das erste längliche Führungselement und damit auf den röhrenförmiger Körper übertragen werden.

Die axial zueinander verschobenen Vorsprünge können in einem Winkel von ca. 90° zueinander verschoben sein. Dann treten die Vorsprünge sehr ausgeprägt nach außen.
Die axial zueinander verschobenen Vorsprünge können auch in einem Winkel von ca. 45° zueinander verschoben sein. Auf diese Weise weist die Oberfläche eine höhere Feinstruktur auf, was für bestimmte Indikationen bei der Implantation gewebeschonender und damit bevorzugt sein kann. Allerdings wirkt sich diese Art der sehr gewebeschonenden Ausbildung der Vorsprünge eventuell etwas mindernd auf die Fixierung vor Ort aus. Es muß daher jeweils abgewogen werden, welche Oberflächenstruktur und damit welche der beiden genannten Ausführungen der Verschiebung, in einem Winkel von 90° oder 45° zueinander, gewählt wird.

Die bei dem Stauchen des ersten Führungselements in axialer Richtung aus der Oberfläche des ersten Führungselements nach außen tretenden Vorsprünge sind unter Bildung von Schenkeln aufgestellt und bilden zueinander einen Winkel α. Dieser ist bevorzugt variabel einstellbar.
Dies kann leicht dadurch erreicht werden, daß das Stauchen des ersten Führungselements stärker oder weniger stark erfolgt. Entsprechend spitzer oder weniger spitz wird der Winkel α ausgebildet.

Auch auf diese Weise kann Einfluß genommen werden auf die Oberflächenstruktur des ersten Führungselements. Ein weniger spitz ausfallender Winkel α bewirkt eine weniger starke Ausprägung der Oberflächenstruktur und umgekehrt.

Dadurch, daß der röhrenförmige Körper bevorzugt als eine röhrenförmiger flexibler Schlauch aus zumindest einem biologisch verträglichen Kunststoff oder Polymer ausgebildet ist, wobei dessen Material ausgewählt ist aus Polyurethanen, Polyetherblockamiden, Polyamiden, Latex, Polyvinylchlorid und/oder Silikon kann er die durch die nach außen tretenden Vorsprünge ausgebildete Struktur abbilden und gleichzeitig eine mögliche Verletzungsgefahr des umliegenden Gewebes durch die Struktur die Vorsprünge verhindern.

Bezüglich des Materials sollen die Polyetherblockamide (PEBA), welche unter dem Markennamen PEBAX® handelsüblich erhältlich sind, besondere Erwähnung finden. Als thermoplastische Elastomere zeichnen sie sich im Vergleich zu anderen thermoplastischen Elastomeren, wie den Polyurethanen, durch eine geringere Dichte aus. Daneben haben sie hervorragende mechanische wie dynamische Eigenschaften. So weisen sie eine hervorragende Elastizität, Schlagzähigkeit und Dauerfestigkeit auf.

Alternativ kann der röhrenförmige Körper als ein sogenannter Schrumpfschlauch aus zumindest einem biologisch verträglichen Thermoplasten ausgebildet sein. Auch dann kann die durch die nach außen tretenden Vorsprünge ausgebildete Struktur gut abgebildet und gleichzeitig eine mögliche Verletzungsgefahr des umliegenden Gewebes durch die Struktur die Vorsprünge verhindert werden.
Als Thermoplasten kommen hier vor allem Polyolefine, Polyvinylchlorid (PVC), Polyvinylidenfluorid (PVDF), Polytetrafluorethylen (PTFE) und/oder Viton in Frage. Mit Viton® ist dabei ein Kunststoff bezeichnet, der von der Firma DuPont im Handel erhältlich ist. Wenn ein solcher Schrumpfschlauch Verwendung findet, können der röhrenförmige Körper und das schlauchartige Halteelement mit dem Metallgitter quasi nahtlos ineinander übergehen. Es ist keine besondere Verbindung z.B. durch ein Markerband oder eine Klemmverbindung vorzusehen.

Wenn der röhrenförmige Körper aus dem Schrumpfschlauch-Material zusätzlich nach der sogenannten "braiding"-Herstellung als Gewebeschlauch mit Metalleinlage in Form z.B. einer Platinvernetzung ausgebildet ist, können die feinen Metalldrähte in das schlauchartige Halteelement übergehen und damit in dem schlauchartigen Halteelement auslaufen. Durch dieses Auslaufen wird zusätzlich das Metallgitter des schlauchartigen Halteelements im Bereich des Übergangs von röhrenförmigem Körper zu schlauchartigem Halteelement vorteilhaft gestärkt. Schrumpfschläuche sind in vielfältiger Ausführung im Handel erhältlich. Es kann zwischen dünnwandigen, mittel- und dickwandigen Schläuchen gewählt werden.

Wenn der röhrenförmige Körper als gewebeverstärkter und/oder gewebeumflochtener flexibler Schlauch ausgebildet ist und eine metallische Verstärkung aufweist, ist es auch möglich, die metallische Verstärkung sowie das reversibel dehn- und stauchbare Metallgitter des schlauchartigen Halteelements als Sandwich zwischen zwei Kunststofflagen auszubilden. Metallische Verstärkung und Metallgitter können dabei ineinander übergehen.

Vorzugsweise ist der röhrenförmige flexible Schlauch zusätzlich mit einer hydrophilen Beschichtung versehen. Mit dieser hydrophilen Beschichtung wird die Gleitfähigkeit der Schlauchoberfläche erhöht, was einerseits die Implantation erleichtert und auf der anderen Seite dazu beiträgt, den Tragekomfort der implantierten Vorrichtung zu erhöhen.

Es kann außerdem vorgesehen sein, daß der röhrenförmige Körper eine antimikrobielle Silberbeschichtung oder eine Beschichtung aus diamantähnlichem Kohlenstoff (diamond-like carbon oder DLC) aufweist. Diese Beschichtungen sind hauchdünn und dienen jeweils dazu, die Keimbesiedlung zu minimieren.

Der röhrenförmige Körper kann auch als flexibler, gewebeverstärkter und/oder gewebeumflochtener Schlauch ausgebildet sein, wobei eine einfache oder eine mehrfache Gewebeeinlage vorhanden ist.

Solche gewebeverstärkten und/oder gewebeumflochtenen flexiblen Schläuche können bevorzug sein, um eine verbesserte Kraftübertragung zu erzielen. Hier sind beispielsweise Silikonschläuche mit monofiler Polyestergewebeeinlage zu nennen. Es kann auch ein peroxid-vernetztes, monofiles Polyestergewebe verwendet werden. Anstelle von Polyester ist auch ein anderer Kunststoff, wie PEBA geeignet. Bei der Gewebeeinlage kann es sich um eine einfache oder eine mehrfache Gewebeeinlage handeln.

Besonders bevorzugt ist das schlauchartige Halteelement mit Luft unter Normalen- bzw. Atmosphärendruck befüllt. Dadurch können in einfacher Weise viele Schwierigkeiten der Bereitstellung einer Vorrichtung zur Verhinderung von Inkontinenz ausgeräumt werden, die ein mit Flüssigkeit befüllbares Halteelement vorsieht. Der Druck, den die erfindungsgemäße Vorrichtung, bzw. der jeweilige Teil der erfindungsgemäßen Vorrichtung gemäß den anatomischen Gegebenheiten des Patienten auf die Harnröhre ausüben soll, um dessen Inkontinenz wirksam zu verhindern, wird durch Dehnen oder Stauchen des schlauchartigen Halteelements eingestellt. Dieses Dehnen oder Stauchen hat quasi die Wirkung einer Feder.

Das Metallgitter des schlauchartigen Halteelements kann als Rautenmuster ausgebildet sein, wobei die Rauten in gestauchtem Zustand als Schlitze ausgebildet sind, die eine Erstreckung in Längsrichtung des flexiblen Schlauchs aufweisen. Besonders bevorzugt weisen die Schlitze, und damit die Rauten des Metallgitters, eine unterschiedliche Größe auf. Damit kann die Streßbelastung auf das Gittermaterial im Hinblick auf die nachjustierbare und die aktiv justierbare Ausführungsform deutlich reduziert und damit die Haltbarkeit des Materials im Hinblick auf die Langzeitanwendung optimiert werden.

Wenn der Teil der erfindungsgemäßen Vorrichtung justierbar ausgebildet ist, mündet das erste längliche Führungselement an seinem distalen Endbereich in ein Stellglied, und überragt dieses für die Zwecke einer manuellen Justierung oder zur Justierung über die Verbindung mit einem Motor als Antrieb.

Als eine Alternative zu der manuellen Justierung ohne Motor ist es ebenso möglich, die Justierung magnetgesteuert vorzunehmen. Diese Art der Justierung bietet den Vorteil, daß sie von außen, ohne einen Eingriff über die Haut erfolgen kann.

Wenn der Teil der erfindungsgemäßen Vorrichtung aktiv justierbar ausgebildet ist, kann das erste längliche Führungselement als Zahnstange ausgebildet sein, die über die Vorsprünge in dem röhrenförmigen Körper gehalten und über eine Hubvorrichtung mechanisch von Hand oder motorgetrieben bewegbar ist.

Die aktiv justierbare Vorrichtung kann mit einen Bewegungs-, Inklinations-, Druck- und/oder Volumensensor gekoppelt und von zumindest einem dieser Sensoren gesteuert sein.

Mit den erfindungsgemäß einsetzbaren Sensortypen werden die wesentlichen externen wie internen Faktoren einer Überprüfung und Steuerung zugänglich gemacht, welche den ordnungsgemäßen Einsatz oder die Funktionstüchtigkeit der Vorrichtung zur Verhinderung von Inkontinenz beeinflussen können. Dabei können sowohl externe Größen, wie beispielsweise der barometrische Druck, als auch interne Größen, d.h. solche, die physiologisch begründet sind, beispielsweise über den Volumensensor abgefragt werden.
Alle genannten Sensortypen sind im Bereich der Medizintechnik bereits bekannt und werden in verschiedenen Applikationen eingesetzt. Vorteilhaft ist, daß sie äußerlich angebracht werden und damit den Implantationsort nicht weiter belasten.
Da die in dem schlauchartigen Halteelement ausgebildete Luftsäule einen Atmosphärendruck aufweist, ist sie entsprechend den üblichen Luftdruckschwankungen ausgesetzt. Extremere Bedingungen, wie Luftdruckveränderungen bei Flügen oder schon in Höhenlagen über zumindest 2000 m, können durchaus eine Veränderung bewirken. Dem trägt das Überwachen der erfindungsgemäßen Vorrichtung bzw. des jeweiligen teils der Vorrichtung Rechnung. Dabei können die genannten Sensoren zusammen oder alternativ zumindest einer oder zwei davon nach Sinn und Zweck ausgewählt eingesetzt werden.

Die bisher beschriebenen Ausführungsformen der erfindungsgemäßen Vorrichtung, einschließlich der genannten Varianten, betreffen jeweils einen Teil der Vorrichtung zur Verhinderung von Inkontinenz. Die fertige Vorrichtung weist zwei gleiche oder verschiedene Teile der hier vorgestellten Teile der Vorrichtung auf. Diese sind jeweils benachbart zu der Harnröhre eines Patienten implantiert.

Jede der beschriebenen Ausführungsformen der erfindungsgemäßen (Teil-) Vorrichtungen kann nun so in einen menschlichen Körper implantiert werden, daß sie im Doppel oder mit einem anderen der schon zuvor beschriebenen Teile der Vorrichtung kombiniert wird.

Die fertige Vorrichtung zur Verhinderung von Inkontinenz kann somit ganz unterschiedlich aufgebaut sein. Eine Möglichkeit besteht darin, daß zwei Teile der nachjustierbaren und/oder der aktiv nachjustierbaren Vorrichtung, wie sie in Einzelheiten weiter oben beschreiben worden sind, benachbart zu der Harnröhre eines Patienten implantiert sind und so die fertige Vorrichtung bilden.

Eine andere Möglichkeit besteht darin, daß ein Teil der Vorrichtung die nachjustierbare und/oder aktiv nachjustierbare Variante betrifft, während der andere Teil der Vorrichtung in Form der nicht nachjustierbaren Variante ausgewählt ist, und die beiden nun voneinander verschiedenen Teile unter Bildung der fertigen Vorrichtung benachbart zu der Harnröhre eines Patienten implantiert sind.

Selbstverständlich ist es auch möglich, daß die fertige Vorrichtung zwei Teile einer nicht nachjustierbaren Variante der erfindungsgemäßen Vorrichtung aufweisen.

Im folgenden soll die Erfindung anhand von Ausführungsbeispielen und der beigefügten Zeichnung näher erläutert werden.

Es zeigen:
- Fig. 1: eine schematische, geschnittene Ansicht einer Vorrichtung zur Verhinderung von Inkontinenz in einer ersten Ausführungsform, mit einem ersten länglichen Führungselement, das Vorsprünge in expandiertem Zustand aufweist,
- Fig. 2: eine schematische, geschnittene und unvollständig dargestellte Ansicht des ersten länglichen Führungselements mit Schlitzen,
- Fig. 3a: eine schematische, perspektivische Detailansicht von zwei einander gegenüberliegenden, nach außen ragenden Vorsprüngen mit Distanzstück,
- Fig. 3b: eine schematische, geschnittene Detailansicht von zwei einander gegenüberliegenden, nach außen ragenden Vorsprüngen mit Distanzstück,
- Fig. 4: eine schematische, geschnittene Ansicht einer Vorrichtung zur Verhinderung von Inkontinenz in leicht gekrümmter Ausführung und mit zusätzlicher Nachjustierung,
- Fig. 5: eine schematische, geschnittene Ansicht des Metallgitters in nicht expandierter Form als Detailansicht nach Ausschnitt I der daneben dargestellten Gesamtvorrichtung des ersten Ausführungsbeispiels,
- Fig. 6: eine schematische, geschnittene Detailansicht der erfindungsgemäßen Vorrichtung, welche die Verbindung des flexiblen Schlauches mit dem Übergang zu dem Käfig darstellt,
- Fig. 7: eine schematische, geschnittene Ansicht von zwei justierbaren erfindungsgemäßen (Teil-) Vorrichtungen nach der Transplantation in ein Körperlumen,
- Fig. 8a: eine schematische, geschnittene und unvollständig dargestellte Ansicht einer Vorrichtung zur Verhinderung von Inkontinenz in einer dritten, aktiv justierbaren Ausführungsform, welche das Stellglied als Justiereinrichtung zeigt,
- Fig. 8b: eine schematische, geschnittene Detailansicht der erfindungsgemäßen Vorrichtung nach Fig. 8a, welche die Verbindung des flexiblen Schlauches mit dem Übergang zu dem Käfig darstellt,
- Fig. 8c: eine schematische, geschnittene Detailansicht der erfindungsgemäßen Vorrichtung nach Fig. 8a, welche die äußere Hülse mit dem von dieser aufgenommenen Kolben des Stellgliedes als Justiereinrichtung näher darstellt, und
- Fig. 9: eine perspektivisch, dreidimensional dargestellte Teilansicht des ersten länglichen Führungselements mit Vorsprüngen, die axial in einem Winkel von ca. 45° zueinander verschoben sind.

### 1. Ausführungsbeispiel: Justierbare Vorrichtung

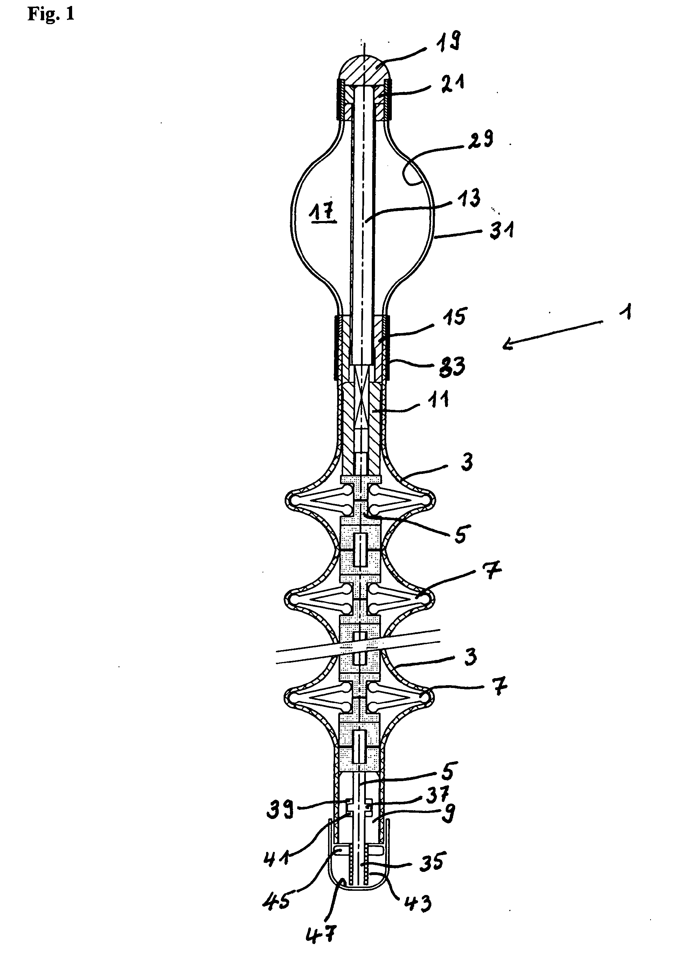

In Fig. 1 ist eine insgesamt mit der Bezugsziffer 1 bezeichnete Vorrichtung zur Verhinderung von Inkontinenz bzw. der eine Teil einer solchen Vorrichtung 1 dargestellt, welcher dann im implantierten Zustand mit einem entsprechenden zweiten Teil der Vorrichtung 1 ergänzt und diese beiden Teile der Vorrichtung 1 dann beidseitig der Harnröhre platziert werden. Zur Vereinfachung wird allerdings bei den nachfolgenden Erläuterungen der eine Teil der Vorrichtung 1, der also nur einen Teil der verwendungs- oder gebrauchsfertigen Vorrichtung 1 darstellt, insgesamt als die Vorrichtung 1 bezeichnet.

In ihrem grundsätzlichen Aufbau und ihrer Funktionsweise entspricht die Vorrichtung 1, wie sie nachfolgend beschrieben wird, derselben Vorrichtung 1 des ersten Ausführungsbeispieles der PCT/EP2010/003757, worauf zusätzlich vollinhaltlich Bezug genommen wird. Die grundsätzliche Funktionsweise der Vorrichtung 1 dieses ersten Ausführungsbeispieles wird jedoch nachfolgend noch einmal beschrieben, um anschließend die hier vorgenommenen erfindungswesentlichen Änderungen zu erläutern.
Entsprechend weist die Vorrichtung 1 einen röhrenförmigen Körper 3 auf, der als ein flexibler Schlauch ausgebildet ist. Der röhrenförmige Körper 3 ist aus einem biologisch verträglichen Kunststoff oder allgemein Polymer gebildet, so daß er in einen menschlichen Körper implantiert werden kann. Bei der Erprobung haben sich in Vorversuchen ein Polyetherblockamid (PEBA), das unter dem Markennamen PEBAX® handelsüblich erhältlich ist, Latex, Polyvinylchlorid (PVC) und Silikon als gleichermaßen geeignet erwiesen. Dem Fachmann sollte dabei ersichtlich sein, daß mit diesen Kunststoffen bzw. Polymeren beispielhaft verwendbare Materialien angegeben worden sind, ohne daß dies einschränkend zu verstehen ist. In bezug auf Latex ist anzumerken, daß sich neben Naturkautschuk auch Synthesekautschuke eignen.

Bei Vorversuchen wurden dieselben Materialien getestet, welche schon in dem 1. Ausführungsbeispiel gemäß der PCT/EP2010/003757 herangezogen worden sind. Neu hinzugekommen ist, daß zunächst ein Silikonschlauch ohne weitere Verstärkung getestet wurde. Dann wurde gemäß der PCT/EP2010/003757 ein Silikonschlauch verwendet, der eine monofile Polyestergewebeeinlage aufwies. Bei einem weiteren Vorversuch wurde derselbe Schlauch, jedoch in platinvernetzter Ausführung verwendet. Damit konnte eine Härte von ca. 70° Shore A erzielt werden.
Noch ein weiterer Vorversuch wurde mit einem peroxid-vernetzten, monofilen Polyestergewebe als Verstärkung des Silikonschlauches durchgeführt.
Die Verstärkung durch die Gewebeeinlage und die Platinvernetzung hatten gemäß der PCT/EP2010/003757 den Zweck, eine optimierte Kraftübertragung zu ermöglichen. Dies schien hier aus den noch zu erläuternden Gründen nicht mehr wesentlich im Vordergrund zu stehen. Da aber zu erwarten ist, daß die verstärkenden Gewebeeinlagen und die Vernetzung zu der Langzeitstabiblität der erfindungsgemäßen Vorrichtung 1 beitragen können, wurden sie vorliegend genauso überprüft. Dabei war es erforderlich, die genannten verstärkenden Materialien noch auf ihre Elastizität zu überprüfen.
Alle Versuche wurden mit guten Ergebnissen in bezug auf die Elastizität, die Haltbarkeit und die Belastbarkeit der eingesetzten Schläuche abgeschlossen. Es ist jedoch festzustellen, daß mit der hier eingesetzten erfindungsgemäßen Vorrichtung, insbesondere der neuartigen, nachfolgend noch zu erläuternden geänderten Gestaltung des röhrenförmigen Körpers 3, auch ein einfacher Silikonschlauch verwendbar ist. Dies ist aus Kostengründen sehr vorteilhaft.

Im Unterschied zu der PCT/EP2010/003757 wird hier ein anderer Weg vorgeschlagen, um den flexiblen Schlauch, der den röhrenförmigen Körper 3 bildet, nach seiner Implantation in Körpergewebe formbeständig zu erhalten. Damit wird erfindungsgemäß gleichzeitig erreicht, daß der röhrenförmigen Körper 3, und damit die gesamte Vorrichtung 1, ortsfest verankert werden kann. Eine solche ortsfeste Verankerung meint, daß die Vorrichtung weder langfristig wandert noch bei kurzfristigen Erschütterungen ihren Platz am Implantationsort verläßt.

Um eine solche ortsfeste Verankerung zu erreichen, weist der röhrenförmige Körper 3 ein erstes längliches Führungselement 5 auf, das aufgrund der an ihm ausgebildeten Vorsprünge 7 gleichzeitig als Fixiereinrichtung dient. Dieses erste längliche Führungselement 5 in Form der Fixiereinrichtung durchzieht den flexiblen Schlauch des röhrenförmigen Körpers 3 im wesentlichen über seine gesamte Länge.
Im vorliegenden Ausführungsbeispiel ist das erste längliche Führungselement 5, einschließlich seiner Vorsprünge 7, aus einer Nickel-Titan-Legierung gebildet, welche superelastische Eigenschaften und ein niedriges Elastizitätsmodul aufweist. Wesentlich für diese Art der Legierung und ihre Verwendung im Rahmen des erfindungsgemäßen ersten länglichen Führungselements 5 ist ihre Elastizität und die Ausnutzung ihres Form-Gedächtnis-Effekts (Memory-Effekt). Dieser dient dazu, den Vorsprüngen 7 des ersten länglichen Führungselements 5 unter Aufwendung einer Kraft ihre Form zu geben und sie so geeignet einzustellen. Dies wird weiter unten noch in Einzelheiten ausführlich erläutert.

Das erste längliche Führungselement 5 mündet an dem distalen, d.h. bei einer gedachten Implantation von der jeweiligen Körpermitte weggerichteten Ende des röhrenförmigen Körpers 3, in ein Stellglied 9. Auch dessen Funktion wird aus Gründen der Übersichtlichkeit erst weiter unten, d.h. zu einem späteren Zeitpunkt, erläutert.
An seinem proximalen, d.h. an dem zum Körper des Implantatträgers hin verlaufenden Ende, mündet das erste längliche Führungselement 5 in eine Kupplung 11. Dabei handelt es sich um eine elastische Kupplung 11 aus einem Elastomer. Die Kupplung 11 ist biegeelastisch. Sie dient als Verbindungselement zu einer Gewindestange 13, die am proximalen Ende des röhrenförmigen Körpers 3 mittels einer Gewindemutter 15 gehalten wird. Etwa in diesem Bereich findet auch der Übergang von dem röhrenförmigen Körper 3 zu einem insgesamt mit 17 bezeichneten Käfig statt, der so ausgebildet ist, daß er in Zusammenwirkung mit dem röhrenförmigen Körper 3 die Inkontinenz bei einem Patienten wirksam verhindern kann. Die Gewindestange 13 durchläuft den Käfig 17, der Luft unter Normalendruck aufweist, um in einen Verschlußstopfen 19 zu münden, der den Käfig 17 nach außen hin luft- und flüssigkeitsdicht verschließt und gleichzeitig als Axiallager 21 für die Gewindestange 13 dient.

Nachdem die Vorrichtung 1 auf diese Weise in den Grundzügen ihres grundsätzlichen Aufbaus erläutert worden ist, soll nun näher auf die insbesondere gegenüber der PCT/EP2010/003757 neuartige Gestaltung des ersten länglichen Führungselements 5 eingegangen werden.

Damit jeder der beiden Teile der Vorrichtung zur Verhinderung von Inkontinenz ortsfest an dem Ort verbleibt, wohin er implantiert worden ist und nicht durch z.B. heftigere Bewegungen des Implantat-Trägers in dem Körpergewebe wandert, weist der röhrenförmige Körper 3 die schon genannten Vorsprünge 7 auf. Ihre Ausbildung an dem ersten länglichen Führungselement 5 wird im folgenden näher beschrieben.

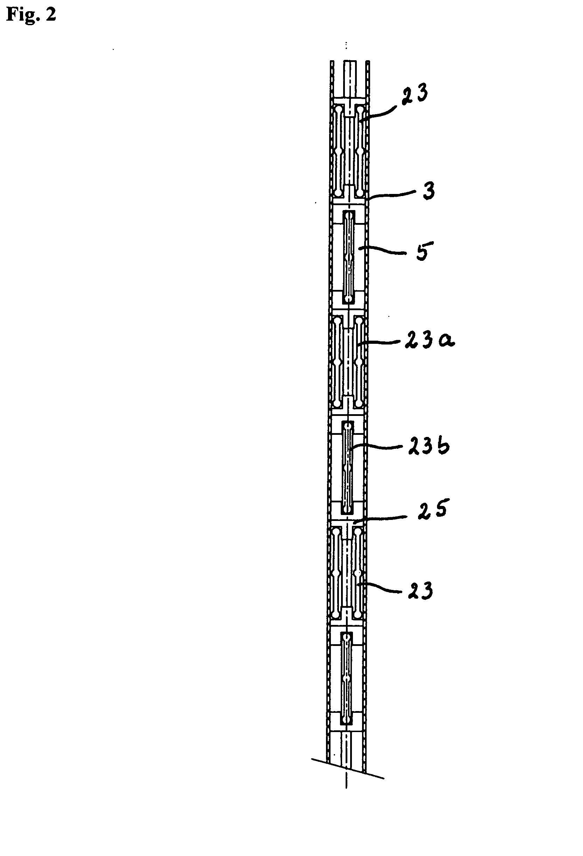

In den Fig. 2a und 2b ist das erste längliche Führungselement 5 dargestellt, das die Vorsprünge 7 in einer nicht expandierten Form zeigt. Das erste längliche Führungselement 5 ist ein Hohlkörper, der hier zunächst an definierten Stellen und in definierten Abständen an der Oberfläche des Hohlkörpers Schlitze 23 aufweist. Dabei ist es für eine ausreichende Fixierung des länglichen Führungselementes 5 und damit der gesamten Vorrichtung 1 von Bedeutung, daß sich Bereiche mit unterschiedlichen Anordnungen der Schlitze 23a, 23b abwechseln, die in einer unterschiedlichen Orientierung der Vorsprünge resultieren. Dieses Abwechseln der Schlitzanordnungen 23a, 23b ist in der Weise zu verstehen, daß die in dem Bereich 23a angeordneten Schlitze 23 relativ zu den in Bereich 23b angeordneten Schlitzen 23, in axialer Richtung gesehen, verschoben sind. Dabei reicht es für eine ortsfeste Fixierung der Vorrichtung 1 aus, wenn in den Bereichen mit unterschiedlichen Anordnungen der Schlitze 23a, 23b insgesamt drei Vorsprünge 7, bevorzugt jedoch vier Vorsprünge 7 gebildet werden. D.h. die beiden Bereiche 23a und 23b weisen insgesamt Schlitze 23 in der Weise auf, daß drei, bevorzugt vier Vorsprünge gebildet werden.
Wenn die beiden Bereiche 23a und 23b insgesamt vier Vorsprünge 7 aufweisen, bedeutet dies gemäß der vorliegend erläuterten Ausführungsform, daß die Vorsprünge 7 des ersten Bereiches 23a gegenüber den Vorsprüngen 7 des zweiten Bereiches 23b um 90°, in axialer Richtung gesehen, verschoben sind. Wie aus den Fig. 2a und 2b ersichtlich, wechseln sich die Bereiche 23a und 23b jeweils im Verlauf der Länge des ersten länglichen Führungselementes 5 ab, sofern an dem ersten länglichen Führungselement 5 Schlitze 23 vorgesehen sind und entsprechend Vorsprünge 9 gebildet werden sollen.

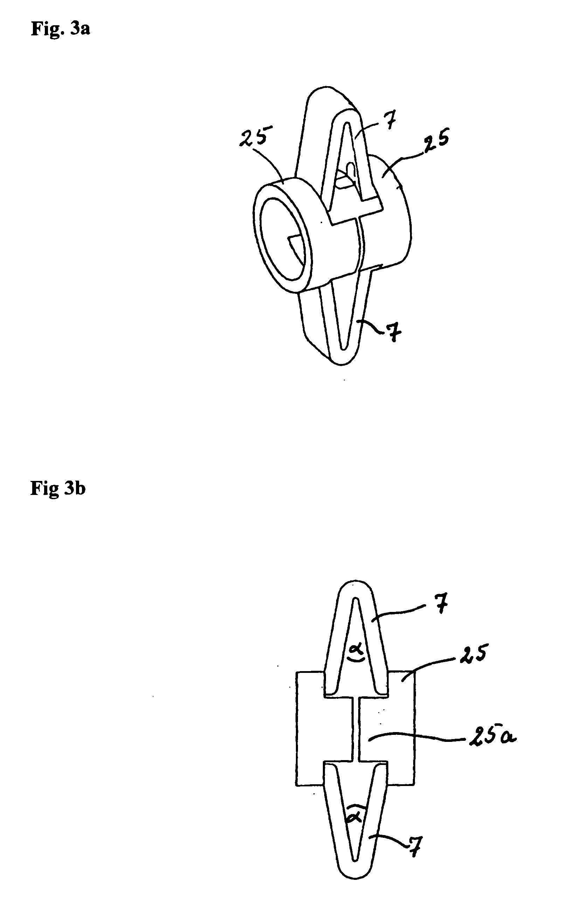

Um ausgehend von den Schlitzen 23 die Vorsprünge 7 zu bilden, wird das erste längliche Führungselement in axialer Richtung gestaucht. Dadurch stellen oder klappen sich die durch die Bildung der Schlitze 23 vordefinierten, aber noch nicht aus der Oberfläche des röhrenförmigen ersten länglichen Führungselementes 5 herausragenden Vorsprünge 7 auf, treten damit nach außen und ergeben die aufgestellten fertigen Vorsprünge 7.
Um diese Vorsprünge 7 in einer Weise voneinander zu beabstanden, daß sich dadurch geeignete Verankerungen für die Vorrichtung 1 in dem jeweiligen Körper bilden, in den implantiert wird, sind Teilbereiche 25 vorgesehen, die keinen Schlitz aufweisen und zusätzlich Distanzstücke 25a, durch welche der Winkel α eingestellt werden kann, in dem sich die Vorsprünge 7 bei dem Herausragen aus der Oberfläche des ersten länglichen Führungselementes 5 aufstellen. In Fig. 3a ist eine perspektivische Detailansicht von zwei einander gegenüberliegenden und nach außen ragenden Vorsprüngen 7 gezeigt, bei denen das Distanzstück 25a die lichte Weite zwischen den Schenkeln jeweils eines Vorsprunges 7, und damit den zwischen den Schenkeln gebildeten Winkel α, vergrößert. Fig. 3b zeigt dieselbe Ansicht, jedoch geschnitten, so daß hier auch der genannte Winkel α angegeben werden kann. Während das Distanzstück 25a die lichte Weite zwischen zwei Schenkeln jeweils eines Vorsprunges definiert, wird über die Teilbereiche ohne Schlitz 25 der Abstand von der Schlitzanordnung des ersten Bereichs 23a zu der Schlitzanordnung des zweiten Bereichs 23b bestimmt. Dabei wird für die Zwecke der Fixierung der Vorrichtung zur Verhinderung von Inkontinenz 1 der Abstand zwischen den Schlitzanordnungen des ersten Bereichs 23a und des zweiten Bereichs 23b gerade so gewählt, daß sich wohldefinierte, voneinander unterscheidbare, noppenartige Vorsprünge bilden können. Auf diese Weise bildet sich entlang des ersten länglichen Führungselements eine quasi-noppenartig geprägte, gewölbte Oberflächenstruktur aus, die einen festen Sitz der Vorrichtung an der Implantatstelle gewährleistet. Die Vorsprünge 7 sind dabei gewebeschonend als abgerundete Auskeilungen nach aussen expandiert.

Da es sich bei dem Material, aus dem das erste längliche Führungselement 5 gebildet ist, um eine Form-Gedächtnis-Legierung aus Nickel und Titan handelt, wie sie im Handel unter der Bezeichnung Nitinol® erhältlich ist, und damit besondere Bedingungen für die Materialbearbeitung bestehen, soll im folgenden auch hierauf noch eingegangen werden. Nitinol® weist einen Nickelanteil von etwa 50% auf und ist bis zu etwa 8% pseudoelastisch verformbar.

Für die Bearbeitung einer solchen Form-Gedächtnis-Legierung aus Nickel und Titan hat es sich besonders günstig erwiesen, die Schlitze 23 mittels Lasertechnik in das röhrenförmige erste längliche Führungselement 5 zu schneiden. Bei den zur Verfügung stehenden Lasertechniken ist wiederum insbesondere die kalte Materialbearbeitung hervorzuheben, bei welcher das Schneiden mit Femtosekunden-Laserpulsen hochpräzise durchgeführt werden kann. Eine solche sogenannte kalte Materialbearbeitung ist durch den StarFemto Laser der Firma ROFIN-BAASEL Lasertech GmbH & Co. KG, Starnberg, Deutschland, möglich. Außerdem wird auf die bekannte Technik des Laserätzens verwiesen, mit der ebenfalls aus Nitinol Schlitze 23 geschnitten werden können.

Aufgrund des gewählten Materials einer Form-Gedächtnis-Legierung für das röhrenförmige erste längliche Führungselement 5 ist es möglich, eine an der Achse gekrümmte Form vorzusehen, die es ermöglicht, den örtlichen anatomischen Gegebenheiten im Bereich des Harnleiters, d.h. des Implantationsortes, Rechnung zu tragen. Dabei sind zwei Varianten zu unterscheiden. Zum einen kann das erste längliche Führungselement 5 vor dem Implantieren bereits in die gewünschte Krümmung gebracht werden, indem die Schlitze 23 für die Vorsprünge 7 eine ungleiche Länge aufweisen, die bei dem Expandieren eine Krümmung bewirkt. Das erste längliche Führungselement 5 kann aber auch in seiner expandierten Form durch einen biegsamen Stab, der die gewünschte Krümmung vorgeformt aufweist, in der gewünschten Weise gekrümmt werden. Dies ist sogar noch nach dem Implantieren möglich, da das erste längliche Führungselement 5 als Hohlkörper ausgebildet ist und der Stab dann von außen gebogen und geformt und dann eingeführt werden kann.

Weitere Untersuchungen wurden mit Schlitzen 23 für die Vorsprünge 7 in ungleicher Länge durchgeführt. Dazu wurden die Bereiche 23a, 23b mit Schlitzen 23 versehen, die an den sich gegenüberliegenden Seiten ungleich geschnitten oder geätzt wurden, so daß sich beim Stauchen die Vorsprünge 7 in unterschiedlicher Weise aufstellen, wobei der eine Vorsprung 7 länger ausgebildet ist als der gegenüberliegende andere Vorsprung 7.

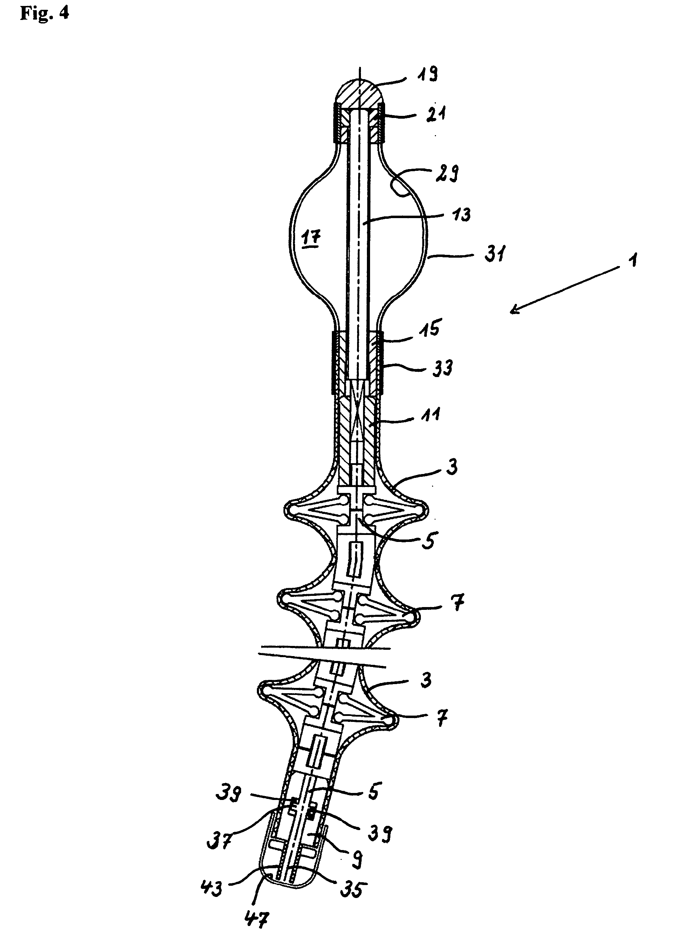

In Fig. 4 ist eine erfindungsgemäße Vorrichtung in leicht gekrümmter Form dargestellt, die zusätzlich ein Nachjustieren vor Ort, d.h. nach dem Implantieren veranschaulicht. Darauf soll im folgenden noch eingegangen werden.

Das Nachjustieren der erfindungsgemäßen Vorrichtung 1 läßt sich durch einen Vergleich zwischen den Figuren 1 und 4 erläutern. Die Fig. 1 und 4 zeigen dabei grundsätzlich ein und dasselbe Vorrichtungsteil 1.
Es wird die Wirkung einer am Stellglied 9 in noch zu beschreibender Weise angreifenden Kraft gezeigt, welche das erste längliche Führungselement 5 bewegt und dabei den Käfig 17 leicht auseinanderzieht. Der Käfig 17 erhält dadurch eine im Vergleich zu Fig. 1 mehr in die Länge gezogene Form. Im implantierten Zustand bedeutet dies, daß der Druck auf die Harnröhre, in deren unmittelbarer Nachbarschaft das Implantat sich im Körper befindet, nachläßt. Der hier ausgenutzte Effekt läßt sich in etwa mit einem Federeffekt vergleichen.

Damit zeigt sich ein wesentlicher Unterschied zwischen der Vorgehensweise, wie im Stand der Technik ein Justieren des dort als Ballon ausgebildeten Halteelements bewirkt wird, gegenüber dem Justieren des erfindungsgemäßen Käfigs. Während der Ballon nach dem Stand der Technik grundsätzlich mit einer Flüssigkeit befüllt ist und z. B. über eine Hohlnadel mit weiterer Flüssigkeit oder einem vergleichbaren Medium versehen wird, um ihn zu erweitern, oder mittels der Hohlnadel Flüssigkeit entzogen wird, wenn ein Verjüngen bzw. Verkleinern erforderlich ist, erfolgt das Justieren erfindungsgemäß über das Verändern einer Luftsäule. Die Ausbildung dieser Luftsäule wird verändert, indem eine Kraft an dem Stellglied 9 angreift, welche auf das erste längliche Führungselement 5 wirkt, dieses zusammen mit der Kupplung 11 bewegt und damit den Käfig 17 - im Beispiel von Fig. 4 - stärker in die Länge zieht. Zwischen der Kupplung 11 und der Gewindemutter 15 entsteht ein Zwischenraum oder Spiel 27.

D.h. über das Stellglied 9 wird eine Kraft auf das erste Führungselement 5 ausgeübt, die dazu führt, daß sich dieses erste Führungselement 5 bewegt, was sich über die Kupplung 11 in der Weise auf den Käfig 17 auswirkt, daß dieser auseinandergezogen wird. Aus dem Vergleich der Fig. 1 und 4 ist ersichtlich, daß der Käfig 17 in Fig. 4 eine mehr in die Länge gezogene Form aufweist, als dies in Fig. 1 der Fall war.

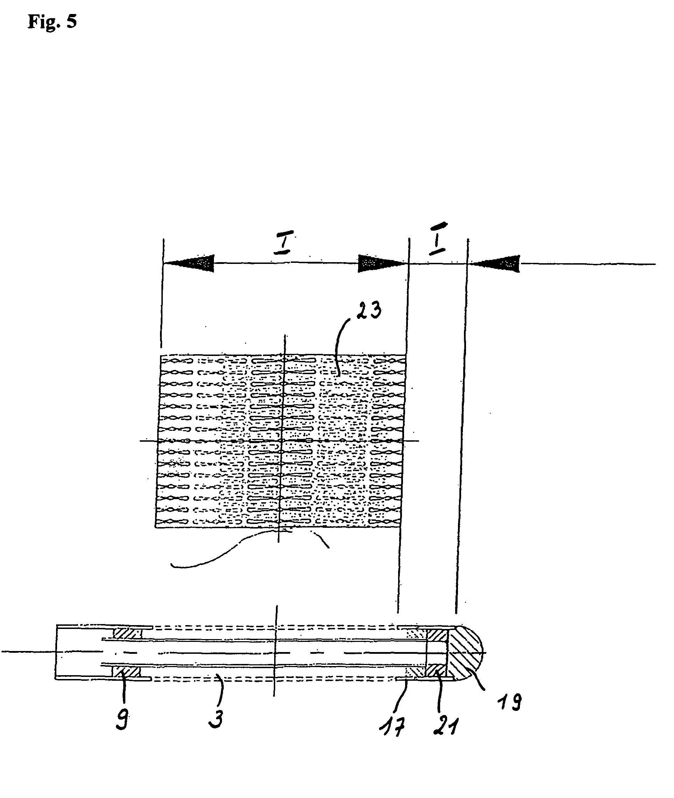

Auch wenn sich der Käfig 17, wie er hier eingesetzt wird, nicht von der Ausführung in der PCT7EP2010/003757 unterscheidet, so daß auf die dortigen Ausführungen vollumfänglich Bezug genommen werden kann, soll er nachfolgend noch einmal erläutert werden.
Der Käfig 17 ist aus einem feinen Metallgitter 29 gebildet, das Fig. 5 zeigt, wie es neuerdings z. B. aus dem Bereich der Karotis für Kathedereingriffe mit Metallgitterstents bekanntgeworden ist und dort verwendet wird. Dabei wird das feine Metallgitter 29 hier als Rautenmuster ausgebildet, da es Kapazität für eine notwendige Aufdehnung bei Expansion des Käfigs 17 aufweisen soll. D.h. durch die Ausbildung in Rautenform gelingt es, den eigentlich nicht dehnbaren, dafür aber sehr beständigen Werkstoff Metall für eine Ausführung nutzbar zu machen, in der ein Expandieren - dann sind die Rauten gedehnt - und Verkleinern - unter Verringern der diagonalen Abstände innerhalb der Rauten - möglich wird. Der Werkstoff Metall wird auf diese Weise flexibel.
In Fig. 5 ist ein Musterausschnitt des Käfigs 17 in nicht expandierter Form dargestellt und die ganze Vorrichtung 1 in nicht expandierter Form noch einmal daneben abgebildet. Aus dieser Abbildung ergibt sich, daß die rautenförmige Gitterstruktur zu Schlitzen zusammengezogen ist, welche sich in Längsrichtung des flexiblen Schlauchs 3 erstrecken. Es hat sich in Versuchen gezeigt, daß die Streßwirkung auf das Material noch weiter optimiert werden kann, wenn die Schlitze, und damit die Rauten des Metallgitters 29, eine unterschiedliche Größe aufweisen.

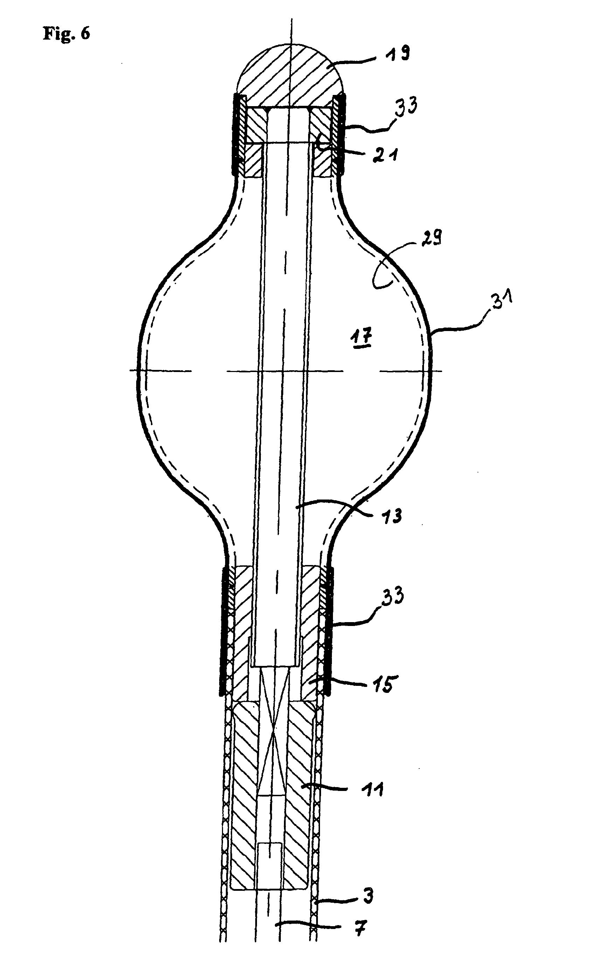

Das Metallgitter 29 des Käfigs 17 ist zusätzlich mit einem Kunststoff luft- und flüssigkeitsdicht überzogen. Im Ausführungsbeispiel wird dies dadurch erreicht, daß eine aus Fig. 6 ersichtliche PTFE-Hülse 31 über das Metallgitter 29 gezogen und über ein in Fig. 6 näher dargestelltes, beidseitig an der Hülse befestigtes Markerband bzw. eine Klemmverbindung 33 gehalten wird. Die Flüssigkeitsabdichtung kann aber auch z. B. mittels Silikonbeschichtung oder einer Beschichtung des Metallkäfigs 17 mit einem vergleichbaren Kunststoff erreicht werden.

Die erfindungsgemäße Vorrichtung zur Verhinderung von Inkontinenz 1, wie sie in den Fig. 1 und Fig. 4 dargestellt und hier näher beschrieben ist, stellt eine nachjustierbare erfindungsgemäße Vorrichtung 1 dar. Dabei kann das Nachjustieren auf unterschiedliche Weise erfolgen. Im einfachsten Fall wird das erste längliche Führungselement 5 manuell vor- oder zurückbewegt, damit wird entsprechend die Kupplung 11 bewegt und der Käfig 17 gestreckt oder gestaucht.
In einer weiteren Ausführungsform weist der hier beschriebene Teil der Vorrichtung 1 einen Motorantrieb auf. Der Motor selbst ist in den Fig. nicht dargestellt.

Da das Stellglied 9 ein Bestandteil der erfindungsgemäßen Vorrichtung zur Verhinderung von Inkontinenz ist, der sich von den Ausführungsformen, wie in der PCT/EP2010/003757 offenbart, unterscheidet, soll darauf im folgenden noch ausführlich eingegangen werden.

Die gemäß der PCT/EP2010/003757 in dem flexiblen Schlauch angebrachte flexible Welle der Vorrichtung zur Verhinderung von Inkontinenz sorgt durch eine Drehbewegung dafür, daß über die am entgegengesetzten, distalen Ende vorgesehene Kupplung eine Kraft auf die Gewindestange ausgeübt wird, die sich in dem Käfig befindet. Dadurch kann dieser in seiner Länge bzw. Ausdehnung gestreckt oder gestaucht werden.

Demgegenüber wird das erste längliche Führungselement 5 gemäß der vorliegenden Erfindung nicht gedreht, sondern führt eine Linearbewegung in axialer Richtung aus. Um dies zu erreichen, weist das Stellglied 9 gegenüber der PCT/EP2010/003757 einen anderen Aufbau auf.
Dazu wird das erste längliche Führungselement 5 an seinem distalen Ende durch das Stellglied 9 hindurchgeführt, so daß es dieses in distaler Position, d.h. nach aussen hin, mit einem Abschnitt 35 überragt. In dem Bereich, der durch das Stellglied 9 hindurchgeführt wird, weist das erste längliche Führungselement 5 keine die Vorsprünge 7 bildenden Schlitze 23 auf. Es ist in diesem Bereich aber mit länglichen Nasen 37 versehen, die an der Oberfläche und einander diametral gegenüberliegend in der Weise ausgebildet sind, daß sie in fast komplementär dazu ausgebildete Aussparungen 39 eingreifen, welche in das Stellglied 9 eingearbeitet sind. Diese Aussparungen 39 sind nur insofern nicht komplementär zu den Nasen 37, als sie diese in ihrer Länge, in axialer Richtung betrachtet, überragen. Dadurch entsteht in axialer Richtung ein Bewegungsspielraum oder Spiel 41, das die in der Regel geringfügigen Justierbewegungen ermöglicht, welche für das paßgenaue Einstellen des Käfigs 17 erforderlich werden können. Dabei ist es unerheblich, ob dieses Einstellen des Käfigs 17 manuell oder über einen mit dem Abschnitt 35 gekoppelten Motor bewirkt wird, der eine exakte Feinregulierung gewährleistet.
Der das Stellglied 9 in distaler Position überragende Abschnitt 35 des ersten länglichen Führungselements 5 ist mit einem Gewinde 43 versehen, auf dem eine Mutter 45 angeordnet ist. Zwischen der Kupplung 11 und dieser an das Stellglied 9 anschlagenden Mutter 45 wird das erste längliche Führungselement 5 ortsfest gehalten.
Wenn der Bedarf einer Justierung oder Nachjustierung der erfindungsgemäßen Vorrichtung darin besteht, daß der Käfig 17 stärker gestaucht werden soll, muß die Mutter 45 zunächst leicht gelöst werden. Das erste längliche Führungselement 5 wird weiter in den flexiblen Schlauch eingeführt, was über das Spiel 41 der Aussparungen 39 am Stellglied 9 möglicht ist. Diese Bewegung wird über die Kupplung 11 auf den Käfig 17 übertragen. Das nur begrenzte Spiel 41 der Aussparungen 39 am Stellglied 9 dient gleichzeitig als Sicherheit, damit ein unbeabsichtigtes, über ein vertretbares Maß hinausgehendes stärkeres Stauchen vermieden wird. Die Mutter 45 muß nach der fertigen Justierung des Käfigs 17 gegebenenfalls noch einmal leicht angezogen werden. Dieses Justieren ist manuell wie motorbetrieben möglich.

Wenn der Käfig 17 gestreckt werden soll, wird das erste längliche Führungselement 5 behutsam aus dem Stellglied herausgezogen. Auch hier verhindert das nur begrenzte Spiel 41 der Aussparungen 39 am Stellglied 9 ein über das Vertretbare hinausgehendes stärkeres Strecken des Käfigs 17. Die Mutter 45 hat in diesem Fall nach der fertigen Justierung des Käfigs 17 ein leichtes Spiel und muß entsprechend angezogen werden. Auch dieses Justieren ist manuell wie motorbetrieben möglich.
Eine Verschlußkappe 47 wird auf das distale Ende der Vorrichtung 1 aufgesteckt und sichert sie dadurch zusätzlich ab. Gegebenenfalls weist die Verschlußkappe 47 in ihrem Deckel innenliegend eine Fassung 49 für den Eingriff des distalen Endes des ersten länglichen Führungselements 5, einschließlich der Mutter 45, auf.

Neben dieser Ausgestaltung des Stellgliedes 9 und seinem Zusammenwirken mit dem distalen Ende des ersten länglichen Führungselements 5, das exemplarisch hier erläutert worden ist, sind weitere Konstruktionen möglich, die ein sicheres Justieren der Vorrichtung gewährleisten.

### Implantation der erfindungsgemäßen Vorrichtung in ein Körperlumen gemäß einer ersten Variante

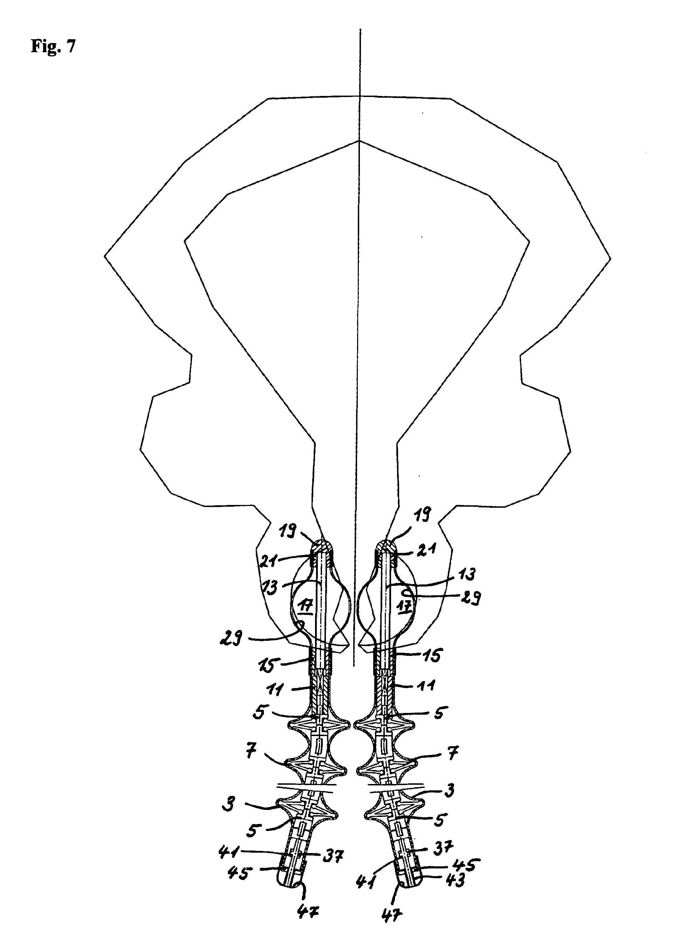

In Fig. 7 ist die Implantation der erfindungsgemäßen justierbaren Vorrichtung 1 zur Verhinderung von Inkontinenz in ein Körperlumen dargestellt. Dabei werden jeweils zwei der Vorrichtungen 1 einander gegenüberliegend so implantiert, daß sie die Harnröhre eines an Harninkontinenz leidenden Patienten mittig zwischen sich halten. Der Käfig 17 in der weiter oben im ersten Ausführungsbeispiel beschriebenen Ausführung ist mit Luft befüllt. Für das Implantieren der beiden Teile der Vorrichtung 1 ist lediglich ein minimal-invasiver Eingriff erforderlich.

Wenn die beiden Vorrichtungen beispielsweise bei einem männlichen Patienten nach einer Prostatektomie verwendet werden, werden sie direkt im Bereich der operierten Prostata implantiert.
Werden die beiden Vorrichtungen beispielsweise bei einem weiblichen Patienten nach einer Hysterektomie verwendet, werden sie direkt im Bereich der Blase implantiert.

Die Ausdehnung der Käfige 17 ist gerade so gewählt, daß bei fertig eingestellter, d.h. justierter Position der beiden Vorrichtungen 17 ein ganz normales Wasserlassen bei einem normalen Blasendruck nicht beeinträchtigt und auch nicht teilweise oder ganz behindert wird. Die Luftsäulen in den beiden Käfigen 17 und ihre Position an der Harnröhre werden über die jeweils proximal mit der Kupplung 11 verbundenen ersten länglichen Führungselemente 5 so eingestellt, daß nur ein willkürliches Wasserlassen, z. B. bei Streßinkontinenz, verhindert wird. Diese kann auftreten, wenn der Patient husten, niesen oder sonstige vergleichbare ruckartige Bewegungen machen muß. Auch das Heben von Lasten kann Streßinkontinenz auslösen.

Die genaue Justierung der Käfige 17 erfolgt über die beiden weiter oben erläuterten, unterschiedlichen Einstellverfahren, d.h. entweder manuell oder motorbetrieben. Bei der manuellen Einstellung kann diese über einen Magneten erfolgen, so daß Gewebe bzw. Haut nicht geöffnet werden muß. Die Justierung erfolgt dann durch die Haut. In jedem Fall wird die Kupplung 11 über das erste längliche Führungselement 5 nach Bedarf vor- oder zurückbewegt und dadurch der Käfig 17 auf einfache Weise gestreckt oder gestaucht. Je nach Ausgestaltung des ersten länglichen Führungselements 5 kann es durch diese Bewegung geschehen, daß auch die Vorsprünge 7 in bezug auf den Winkel α, welchen ihre Schenkel bilden, entweder unter Vergrößerung des Winkels α etwas gestreckt oder unter einem spitzer zulaufenden Winkel α etwas gestaucht werden, so daß sie etwas weiter nach außen ragen. Grundsätzlich sind jedoch die für die Justierung der Größe bzw. Ausdehnung des Käfigs 17 notwendigen Bewegungen sehr gering. Dasselbe gilt für Veränderungen des Winkels α der Schenkel der Vorsprünge 7.
Für die manuelle Bedienung bietet die Mutter 45 eine gute Angriffsfläche für einen entsprechenden paßgenauen Schlüssel. Die Mutter 45 kann unter anderem als Sechs- oder Vierkantmutter ausgebildet sein. Als Schlüssel kann z.B. ein Universalschlüssel verwendet werden.

Im Fall der motorbetriebenen Bedienung wird ein Motor im Bereich des Stellglieds 9 über das Gewinde 43 des Abschnitts 29, d.h. das aus dem Stellglied 9 noch herausragende distale Ende des ersten länglichen Führungselements 5, mit der Vorrichtung 1 verbunden. Über den Motor wird dann das erste längliche Führungselement 5 mit der Kupplung 11 nach Bedarf vor- oder zurückbewegt und dadurch der Käfig 17 auf einfache Weise gestreckt oder gestaucht, wie weiter oben schon beschrieben. Das erste längliche Führungselement 5 ist dann über den Motor direkt angetrieben.

Zur präzisen Feineinstellung haben sich Magnetventile, die mit einem Elektromagneten zusammenwirken, Federbalken oder Piezostellelemente gleichermaßen als geeignet erwiesen, wobei letztere besonders empfindlich sind und eine besonders gute Feineinstellung ermöglichen.

Zum Einstellen der beiden Teile der erfindungsgemäßen Vorrichtung 1 geht man so vor, daß die Teile in einem minimal-invasiven Eingriff eingebracht und die Wunde zunächst zumindest einen Tag offen gelassen wird, was im Rahmen der minimal-invasiven Chirurgie unproblematisch ist. Gegebenenfalls wird auch der Anschluß für den Motor mitimplantiert.

Nachdem die beiden Teile der Vorrichtung 1 zuverlässig eingestellt worden sind, kann die Mutter 45 fest justiert, gegebenenfalls der Motoranschluß entfernt, die Verschlußkappe 47 aufgesetzt und die Wunde geschlossen werden.

### 2. Ausführungsbeispiel: Aktiv bedienbare, justierbare Vorrichtung

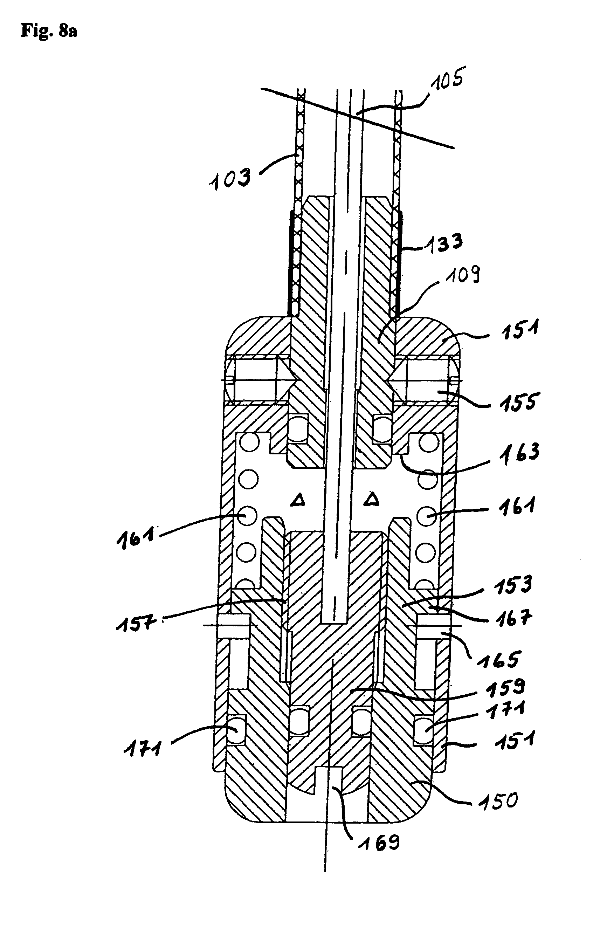

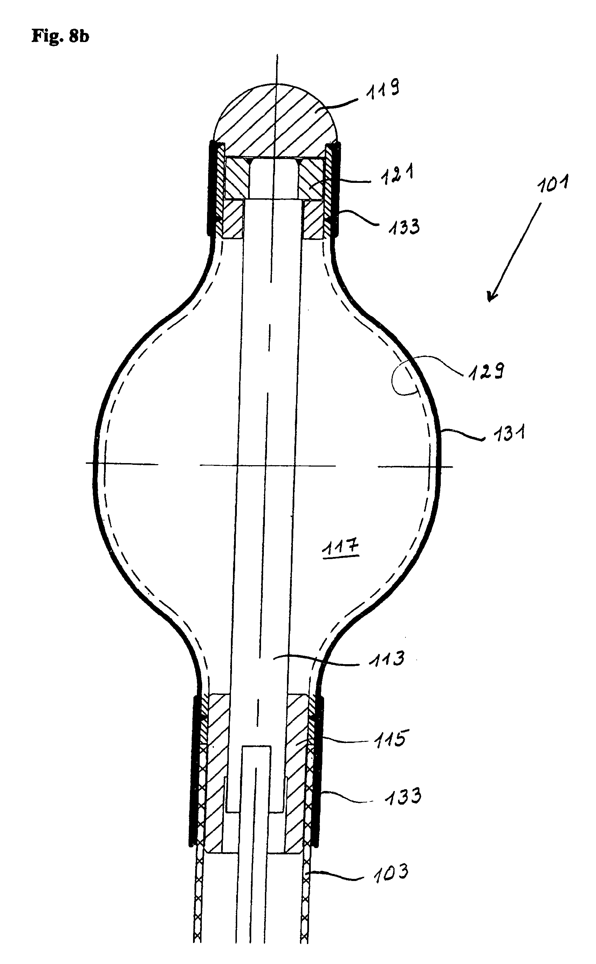

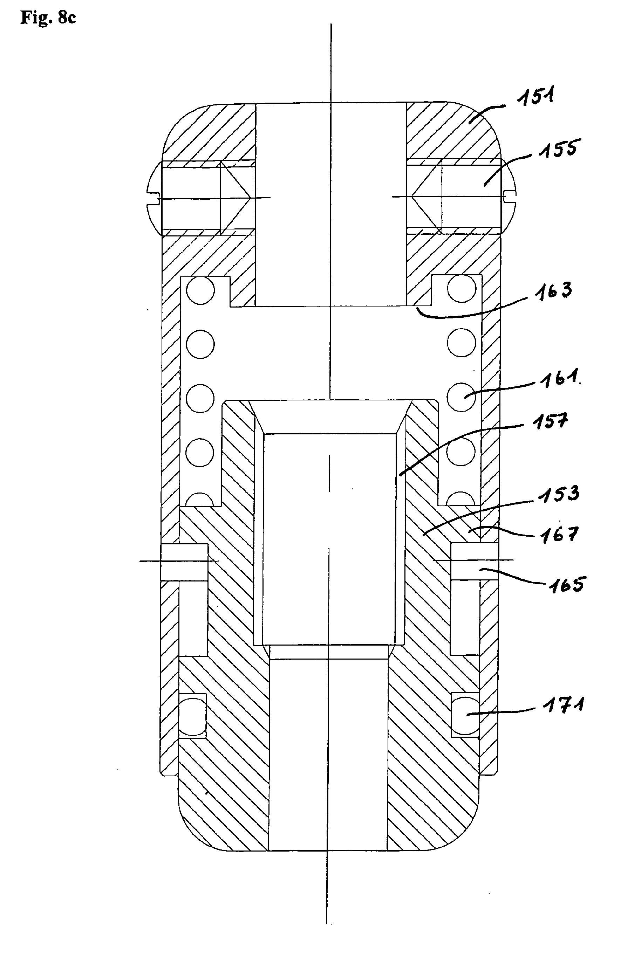

Die Fig. 8a - 8c zeigen eine weitere Ausgestaltung der erfindungsgemäßen Vorrichtung zur Verhinderung von Inkontinenz, die auch nach dem Implantieren weiterhin aktiv bedienbar ist und bleibt. Es werden zu dem ersten Ausführungsbeispiel gleiche Bestandteile mit denselben, jedoch um 100 erweiterten Bezugsziffern versehen. Entsprechend ist die erfindungsgemäße Vorrichtung insgesamt mit der Bezugsziffer 101 bezeichnet.
Die Vorrichtung 101 weist wieder einen röhrenförmigen, flexiblen Schlauch 103 auf, der aus einem biologisch verträglichen Kunststoff oder allgemein Polymer gebildet ist, so daß er in einen menschlichen Körper implantiert werden kann. Insofern wird auf das erste Ausführungsbeispiel verwiesen.
Um seine Formbeständigkeit zu gewährleisten, weist der flexible Schlauch 103 das erste längliche Führungselement 105 auf, an dem die Vorsprünge 7 in der bereits ausführlich beschriebenen Weise gebildet sind. Das erste längliche Führungselement 105 ist als Zahnstange ausgebildet und mündet an dem distalen, d.h. bei einer gedachten Implantation von der jeweiligen Körpermitte weggerichteten Ende des flexiblen Schlauches 103 in das Stellglied 109, das gemäß dieser Variante der erfindungsgemäßen Vorrichtung als eine Justiereinrichtung ausgebildet ist, welche der Patient, in dessen Harnröhrebereich die Vorrichtung implantiert ist, oder der den Patienten betreuende Arzt postoperativ bedienen kann, um die Vorrichtung nachzujustieren.

In der schon in Fig. 6 im Detail gezeigten Weise mündet die Zahnstange an ihrem proximalen, d.h. an dem zum Körper hin verlaufenden Ende, über die Kupplung 11 in die Gewindestange 113, die den Käfig 117 durchläuft und am proximalen Ende des flexiblen Schlauches 103 mittels einer Gewindemutter 115 gehalten wird. Der Übergang von dem flexiblen Schlauch 103 zu dem Käfig 117 ist bereits im ersten Ausführungsbeispiel ausführlich beschrieben worden ist, worauf hier nur verwiesen wird. Der Verschlußstopfen 119 verschließt den Käfig 117 nach außen hin wieder luft- und flüssigkeitsdicht ab und dient gleichzeitig als Axiallager 121 für die Gewindestange.

Als Kunststoff, mit dem das im ersten Ausführungsbeispiel näher beschriebene Metallgitter 129 des Käfigs 117 zusätzlich luft- und flüssigkeitsdicht überzogen ist, wurde in diesem Ausführungsbeispiel eine PTFE-Hülse 131 verwendet, die über das Metallgitter 129 gezogen und über ein beidseitig an der Hülse befestigtes Markerband bzw. eine Klemmverbindung 133 gehalten wird. Die Luft- bzw. Flüssigkeitsabdichtung kann aber auch z. B. mittels Silikonbeschichtung oder einer Beschichtung des Metallkäfigs 117 mit einem vergleichbaren Kunststoff erreicht werden.

Im folgenden soll das als Justiereinrichtung ausgebildete Stellglied 109 der erfindungsgemäßen Vorrichtung zur Verhinderung von Inkontinenz 101 näher beschrieben werden, wie es die Fig. 8a - 8c im Detail zeigen.

Das als aktive Justiereinrichtung ausgebildete Stellglied 109 wird mit seinem proximalen Ende in dem flexiblen Schlauch des länglichen Führungselements 103 an dessen distalem Ende gehalten. Dazu dient ein an dem äußeren Umfang des Schlauchs befestigtes Markerband bzw. eine Klemmverbindung 133. Das genannte proximale Ende des Stellglieds 109 bildet eine Führung 150, die das distale, d.h. von dem Körper weg verlaufenden Ende des als Zahnstange ausgebildeten ersten länglichen Führungselements 105 aufnimmt und so als deren Lagerung dient.
Das Stellglied 109 gemäß dieser Ausführungsform weist des weiteren eine äußere Hülse 151 auf, welche als eine Verbindungshülse von der Führung 150 mit einem Kolben 153 fungiert, und über Schrauben 155 unter üblicher Verwendung von O-Ringen an die Führung 150 geschraubt ist. Über die Schrauben 155 kann die Vorspannung des Käfigs 117 axial eingestellt werden.
Der Kolben 153 ist über ein Gewinde 157 mit einer Halterung 159 verbunden, welche die Zahnstange 105 in ihrem distalen Endbereich aufnimmt.
Zwischen der Führung 150 und der Halterung 159 ist ein deutliches Spiel Δ vorgesehen, daß über Rückstellfedern 161 überbrückt wird, und die Wegstrecke für den möglichen Kolbenhub definiert.
Bei der Bewegung des Kolbens 153 in Richtung auf den Käfig sorgt ein Anschlag 163 im Innern der äußeren Hülse 151 für eine Begrenzung des Kolbenhubs in dieser Richtung. Für eine Begrenzung des Kolbenhubs in entgegengesetzter Richtung ist ein Stift vorgesehen, der in die mit der Bezugsziffer 165 versehenen Ausnehmung eingesetzt wird, und der mit einem entsprechenden Vorsprung 167 an dem äußeren Umfang des Kolbens 153 wechselwirkt, indem der Stift einen Anschlag für den Vorsprung 167 bildet. Dadurch wird vermieden, daß die Führung 150 der Zahnstange 105 in dem Stellglied 109, die über das Gewinde 157 mit dem Kolben 153 durch Verschraubung verbunden ist, ganz aus der äußeren Hülse 151 herausgezogen werden kann. Dichtelemente 171 sorgen des weiteren für eine ausreichende Abgrenzung des Inneren des Stellglieds 109 gegenüber außen.
An der distalen Begrenzung der mit dem Kolben 153 verbundenen Führung 150 der Zahnstange 105, und damit von außen zugänglich, ist eine nutförmige Vertiefung 169 vorgesehen, die als Stellschraube dient, um dort manuell über einen Schraubenzieher die Vorspannung des Käfigs beeinflussen zu können.
Alternativ kann auch bei dieser Ausführungsform anstelle der manuellen, postoperativen Justierung durch den Patienten selbst oder den diesen betreuenden Arzt ein Motor über diesen distalen Bereich des Stellglieds 109 mit der Vorrichtung 101 verbunden werden.

Die in dem Käfig 117 ausgebildete Luftsäule, welche wie in den weiteren, bisher geschilderten Ausführungsformen den üblichen Luftdruck aufweist, ist entsprechend den Luftdruckschwankungen ausgesetzt. Längere Versuchsreihen haben gezeigt, daß diese Luftdruckschwankungen im Normalfall nur eine geringe Veränderung der Luftsäule in dem Käfig bewirken. Extreme oder extremere Bedingungen, wie Luftdruckveränderungen bei Flügen oder schon in Höhenlagen über zumindest 2000 m, können durchaus eine Veränderung bewirken.
Hier wurde zusätzlich vorgesehen, Bewegungs-, Inklinations- und/oder Drucksensoren mit der erfindungsgemäßen Vorrichtung zu kombinieren. Alle drei genannten Sensortypen sind im Bereich der Medizintechnik bereits bekannt und werden in verschiedenen Applikationen eingesetzt. Vorteilhaft ist, daß sie äußerlich angebracht werden und damit den Implantationsort nicht weiter belasten.
In die Vorversuche miteinbezogen wurde auch ein wahlweises Einsetzen der genannten Sensoren je nach Bedarf. Auf Flugreisen hat sich entsprechend der Vorversuche die ausschließliche Verwendung eines Drucksensors als ausreichend erwiesen. Bei normaler Verwendung kann es sehr angenehm sein, zusätzlich einen Bewegungssensor zu verwenden, der z.B. die Ruhephasen des Patienten berücksichtigt und ein Nachjustieren in dieser Zeit unterbindet, indem keine Signale an den Drucksensor weitergeleitet werden. Die Kopplung der Sensoren untereinander ist nach heutiger Meß- und Regeltechnik für den Fachmann möglich, so daß hier nicht gezielt darauf eingegangen werden muß.

Entsprechend ist auch ein vollautomatisches Nachregeln möglich. Dies sollte sogar angestrebt werden, damit nicht der Patient selbst als fachfremde Bedienung der Vorrichtung auftritt, sondern ein Automat über die Stellschraube 169 bzw. den Motor feinjustiert. Regelmäßige Kontrollen des betreuenden Arztes runden dann die Justierung in medizinisch vertretbarer Weise ab.

Bei der bisher beschriebenen (Teil-) Vorrichtung zur Verhinderung von Inkontinenz in einer ihrer Varianten waren die an dem ersten länglichen Führungselement ausgebildeten Vorsprünge jeweils axial zueinander in einem Winkel von ca. 90° verschoben.

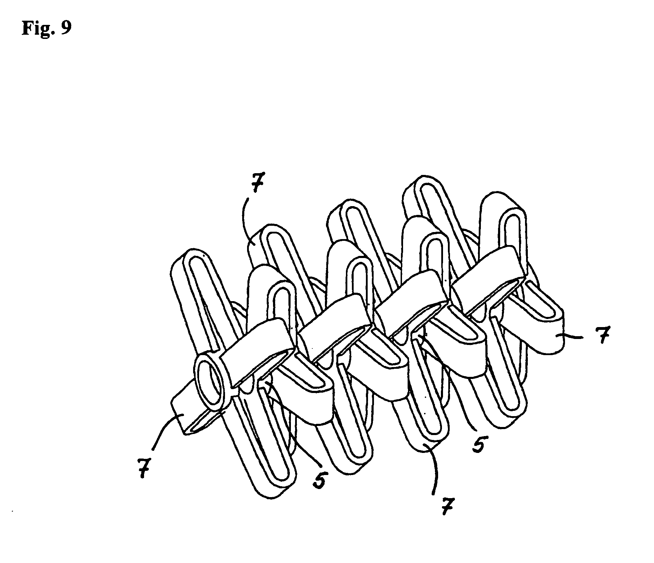

Fig. 9 veranschaulicht demgegenüber ein erstes längliches Führungselement, an welchem die Vorsprünge so ausgebildet sind, daß sie jeweils axial zueinander in einem Winkel von ca. 45° verschoben sind.

Beide Varianten, eine axiale Verschiebung der Vorsprünge zueinander in einem Winkel von 90° wie 45°, haben je nach Vorgeschichte und Beschaffenheit des Implantationsortes ihre Vorteile, so daß es abzuwägen ist, ob eine stärkere Ausprägung der Vorsprünge oder eine maximale Gewebeschonung erreicht werden soll.

## Patentansprüche

1. Teil einer Vorrichtung zur Verhinderung von Inkontinenz mit einer Fixiereinrichtung zur ortsfesten Implantation in Körpergewebe, wobei ein röhrenförmiger Körper (3) in seinem Innern ein erstes längliches Führungselement (5; 105) aufweist, sich an den röhrenförmigen Körper (3) ein schlauchartiges Halteelement anschließt, das in seinem Innern ein zweites längliches Führungselement (13) aufweist, welches in axialer Richtung an seiner dem röhrenförmigen Körper (3) entgegengesetzten Seite in einen Abschluß mündet, und wobei das schlauchartige Halteelement als ein reversibel dehn- und stauchbares Metallgitter (29) ausgebildet ist, das einen polymeren Überzug (31) aufweist, mit dem zusammen es einerseits an dem Abschluß und andererseits in dem Bereich des Übergangs zu dem röhrenförmigen Körper (3) luft- und flüssigkeitsdicht gehalten ist, **dadurch gekennzeichnet, daß** das erste längliche Führungselement (5; 105) die Fixiereinrichtung aufweist, indem es Bereiche mit axial zueinander verschobenen Vorsprüngen (7) definiert, wobei die Vorsprünge (7) im nicht expandierten Zustand als Schlitze (23) ausgebildet sind, welche nach einem Stauchen des ersten Führungselements (5; 105) in axialer Richtung aus der Oberfläche des Führungselements nach außen treten und unter Bildung der fertigen Vorsprünge aufgestellt sind.

2. Teil der Vorrichtung nach Anspruch 1, **dadurch gekennzeichnet, daß** die Schlitze im wesentlichen parallel zur Achse des ersten Führungselements verlaufen.

3. Teil der Vorrichtung nach Anspruch 1 oder 2, **dadurch gekennzeichnet, daß** das erste längliche Führungselement (5; 105) aus einer Nickel-Titan-Legierung mit einem Form-Gedächtnis-Effekt (Memory-Effekt)gebildet ist.

4. Teil der Vorrichtung nach einem der Ansprüche 1 bis 3, **dadurch gekennzeichnet, daß** das erste längliche Führungselement (5; 105) im wesentlichen als Hohlkörper ausgebildet ist.

5. Teil der Vorrichtung nach Anspruch 4, **dadurch gekennzeichnet, daß** in das erste längliche Führungselement (5; 105) ein an den Innendurchmesser des Hohlkörpers angepaßter Stab eingeführt ist, der eine vorherbestimmte Krümmung aufweist, die an die Anatomie des Implantationsortes angepaßt ist und die Krümmung auf das erste längliche Führungselement (5; 105) und damit auf den röhrenförmiger Körper (3) überträgt.

6. Teil der Vorrichtung nach einem der Ansprüche 1 bis 5, **dadurch gekennzeichnet, daß** die axial zueinander verschobenen Vorsprünge (7) in einem Winkel von ca. 90° zueinander verschoben sind.

7. Teil der Vorrichtung nach einem der Ansprüche 1 bis 5, **dadurch gekennzeichnet, daß** die axial zueinander verschobenen Vorsprünge (7) in einem Winkel von ca. 45° zueinander verschoben sind.

8. Teil der Vorrichtung nach einem der Ansprüche 1 bis 7, **dadurch gekennzeichnet , daß** die bei dem Stauchen des ersten Führungselements (5; 105) in axialer Richtung aus der Oberfläche des Führungselements nach außen tretenden Vorsprünge (7) unter Bildung von Schenkeln aufgestellt sind, die zueinander einen Winkel α bilden, der variabel einstellbar ist.

9. Teil der Vorrichtung nach einem der Ansprüche 1 bis 8, **dadurch gekennzeichnet, daß** der röhrenförmige Körper (3) als röhrenförmiger, flexibler Schlauch aus zumindest einem biologisch verträglichen Kunststoff oder Polymer ausgebildet ist, wobei das Material ausgewählt ist aus Polyurethanen, Polyetherblockamiden, Polyamiden, Latex, Polyvinylchlorid und/oder Silikon.

10. Teil der Vorrichtung nach einem der Ansprüche 1 bis 8, **dadurch gekennzeichnet, daß** der röhrenförmige Körper (3) als röhrenförmiger Schrumpfschlauch aus zumindest einem biologisch verträglichen Thermoplasten ausgebildet ist, wobei das Material ausgewählt ist aus Polyolefinen, Polyvinylchlorid, Polyvinylidenfluorid, Polytetrafluorethylen und/oder Viton, und daß dieser Schlauch und das schlauchartige Halteelement mit dem Metallgitter (23) ineinander übergehen.

11. Teil der Vorrichtung nach einem der Ansprüche 1 bis 10, **dadurch gekennzeichnet, daß** der röhrenförmige Körper (3) als röhrenförmiger, flexibler Schlauch ausgebildet und mit einer hydrophilen Beschichtung versehen ist.

12. Teil der Vorrichtung nach einem der Ansprüche 1 bis 11, **dadurch gekennzeichnet, daß** der röhrenförmige Körper (3) als flexibler, gewebeverstärkter und/oder gewebeumflochtener Schlauch ausgebildet ist, wobei eine einfache oder eine mehrfache Gewebeeinlage vorhanden ist.

13. Teil der Vorrichtung nach Anspruch 12, **dadurch gekennzeichnet, daß** der röhrenförmiger Schrumpfschlauch eine metallische Verstärkung aufweist, und daß die metallische Verstärkung sowie das reversibel dehn- und stauchbare Metallgitter des schlauchartigen Halteelements als Sandwich zwischen zwei Kunststofflagen ausgebildet sind.

14. Teil der Vorrichtung nach einem der Ansprüche 1 bis 13, **dadurch gekennzeichnet, daß** der röhrenförmige Körper (3) eine antimikrobielle Silberbeschichtung oder eine Beschichtung aus diamantähnlichem Kohlenstoff aufweist.

15. Teil der Vorrichtung nach einem der Ansprüche 1 bis 14, **dadurch gekennzeichnet, daß** das schlauchartige Halteelement mit Luft unter Atmosphärendruck befüllt ist.

16. Teil der Vorrichtung nach einem der Ansprüche 1 bis 15, **dadurch gekennzeichnet, daß** das Metallgitter (29) als Rautenmuster ausgebildet ist, wobei die Rauten in gestauchtem Zustand als Schlitze ausgebildet sind, die vorzugsweise eine Erstreckung in Längsrichtung des flexiblen Schlauchs aufweisen.

17. Teil der Vorrichtung nach einem der Ansprüche 1 bis 16, **dadurch gekennzeichnet, daß** das erste längliche Führungselement (5; 105) an seinem distalen Endbereich in ein Stellglied (9) mündet und dieses zur manuellen Justierung oder zur Justierung über die Verbindung mit einem Motor als Antrieb überragt.

18. Teil der Vorrichtung nach einem der Ansprüche 1 bis 17, **dadurch gekennzeichnet, daß** er aktiv justierbar ausgebildet ist, und daß das erste längliche Führungselement als Zahnstange ausgebildet ist, die über die Vorsprünge (7) in dem röhrenförmigen Körper (3) gehalten und über eine HubVorrichtung mechanisch von Hand oder motorgetrieben bewegbar ist.

19. Teil der Vorrichtung nach Anspruch 17, **dadurch gekennzeichnet, daß** die aktiv justierbare Vorrichtung mit einen Bewegungs-, Inklinations-, Druck- und/oder Volumensensor gekoppelt und von zumindest einem dieser Sensoren gesteuert ist.

20. Vorrichtung zur Verhinderung von Inkontinenz, welche zwei gleiche oder verschiedene Teile der Vorrichtung nach einem der Ansprüche 1 bis 19 aufweist, die jeweils benachbart zu der Harnröhre eines Patienten implantiert sind.

21. Vorrichtung zur Verhinderung von Inkontinenz nach Anspruch 18, welche zumindest ein Teil der justierbaren Vorrichtung gemäß einem der Ansprüche 1 bis 16 und/oder zumindest ein Teil der Vorrichtung gemäß Anspruch 17 und/oder zumindest ein Teil der aktiv justierbaren Vorrichtung gemäß einem der Ansprüche 18 bis 20 aufweist.

## Claims

1. Part of a device for preventing incontinence that has a fastening device for its stationary implantation in the body tissue, with a tube-shaped body (3) inside of which there is a first longitudinal guide element (5; 105), while a hose-like retaining element connects to the tube-shaped body (3), inside of which there is a second longitudinal guide element (13), that opens in the axial direction into a termination on the side thereof opposite the tube-shaped body (3), and where the hose-like retaining element is implemented as a reversible stretchable and collapsible metal mesh (29) fitted with a polymer coating (31), in combination with which it is kept airtight and fluid-tight firstly at the termination and secondly in the region of the transition to the tube-shaped body (3); **characterised in that** the first longitudinal guide element (5; 105) is fitted with the said fastening device, formed by sections with protrusions (7) that are axially displaced with respect to one another, and where the protrusions (7) are shaped as slits (23) when in their non-expanded state, which then extend out from the surface of the guide element when the first guide element (5; 105) is collapsed in the axial direction, thereby raising up the final protrusions.

2. Part of the device according to Claim 1, **characterised in that** the slits basically run parallel to the axis of the first guide element.

3. Part of the device according to Claim 1 or 2, **characterised in that** the first longitudinal guide element (5; 105) is made of a nickel-titanium alloy that has a shape-memory effect.

4. Part of the device according to one of the Claims 1 to 3, **characterised in that** the first longitudinal guide element (5; 105) is basically designed as a hollow body.

5. Part of the device according to Claim 4, **characterised in that** inside the first longitudinal guide element (5; 105) there is inserted a rod that fits the inner diameter of the hollow body, and which has a predetermined curve that matches the anatomy at the place of implantation, and the curve is conveyed to the first longitudinal guide element (5; 105) and therefore to the tube-shaped body (3).

6. Part of the device according to one of the Claims 1 to 5, **characterised in that** the protrusions (7) that are axially displaced in relation to each other are displaced in relation to each other at an angle of approx. 90°.

7. Part of the device according to one of the Claims 1 to 5, **characterised in that** the protrusions (7) that are axially displaced in relation to each other are displaced in relation to each other at an angle of approx, 45°.

8. Part of the device according to one of the Claims 1 to 7, **characterised in that** the protrusions (7) that rise from the surface of the guide element when the first guide element (5; 105) is collapsed in the axial direction, are raised up in such a way that flanks are formed at an angle α to each other, this angle being variable and adjustable.

9. Part of the device according to one of the Claims 1 to 8, **characterised in that** the tube-shaped body (3) is designed as a tube-shaped, flexible hose made of at least one biologically compatible plastic or polymer, the material being selected from among polyurethanes, polyether block amides, polyamides, latex, polyvinyl chloride and/or silicone.

10. Part of the device according to one of the Claims 1 to 8, **characterised in that** the tube-shaped body (3) is designed as a tube-shaped shrink-hose made of at least one biologically compatible thermoplastic, where the material is selected from among polyolefins, polyvinyl chloride, polyvinylidene fluoride, polytetrafluorethylene and/or Viton, and that this hose and the hose-like retaining element with the metal mesh (23) fit into each other.

11. Part of the device according to one of the Claims 1 to 10, **characterised in that** the tube-shaped body (3) is designed as a tube-shaped, flexible hose and has a hydrophilic coating.

12. Part of the device according to one of the Claims 1 to 11, **characterised in that** the tube-shaped body (3) is designed as a flexible, fabric-reinforced and/or fabric-braided hose, and that a single or multiple fabric insert is provided.

13. Part of the device according to Claim 12, **characterised in that** the tube-shaped shrink hose has a metallic reinforcement, and that the metallic reinforcement, as well as the reversible stretchable and collapsible metal mesh of the hose-like retaining element are constructed as a sandwich between two plastic layers.

14. Part of the device according to one of the Claims 1 to 13, **characterised in that** the tube-shaped body (3) has an anti-microbial silver coating or a coating of diamond-like carbon.

15. Part of the device according to one of the Claims 1 to 14, **characterised in that** the hose-shaped retaining element is filled with air at atmospheric pressure.

16. Part of the device according to one of the Claims 1 to 15, **characterised in that** the metal mesh (29) is formed as a diamond pattern, where the rhombuses are formed as slits when in the collapsed state, which are preferably extended in the lengthwise direction of the flexible hose.

17. Part of the device according to one of the Claims 1 to 16, **characterised in that** the first longitudinal guide element (5; 105) opens at its distal end section into a control element (9), which protrudes so that it can be used for manual adjustment or for adjusting via the connection to a motor as drive unit.

18. Part of the device according to one of the Claims 1 to 17, **characterised in that** it is formed so as to be actively adjustable, and that the first longitudinal guide element is formed as a toothed rod, which is held in the tube-shaped body (3) by protrusions (7), and which can be moved via a lifting device mechanically by hand or motor-driven.

19. Part of the device according to Claim 17, **characterised in that** the actively adjustable device is coupled to a motion, inclination, pressure and/or volumetric sensor, and controlled by at least one of these sensors.

20. Device for preventing incontinence which has two identical or dissimilar parts of the device according to one of the Claims 1 to 19, which are each implanted adjacent to the urethra of a patient.

21. Device for preventing incontinence according to Claim 18, which has at least one part of the adjustable device according to one of the Claims 1 to 16, and/or at least one part of the device according to Claim 17, and/or at least one part of the actively adjustable device according to one of the Claims 18 to 20.

## Revendications

1. Partie d'un dispositif pour empêcher l'incontinence, comportant un moyen de fixation pour l'implantation fixe dans du tissu corporel un corps tubulaire (3) présentant dans son intérieur un premier élément de guidage allongé (5 ; 105), un élément de retenue de type tuyau se raccordant au corps tubulaire (3), lequel présente dans son intérieur un second élément de guidage allongé (13) qui débouche, dans la direction axiale, de son côté opposé au corps tubulaire (3), dans un élément terminal, l'élément de retenue de type tuyau étant réalisé sous la forme d'une grille métallique (29) extensible et compressible de manière réversible, qui présente un revêtement polymère (31), par laquelle il est maintenu d'une manière étanche à l'air et aux liquides d'une part à l'élément terminal et d'autre part dans la zone de la transition avec le corps tubulaire (3), **caractérisée en ce que** le premier élément de guidage allongé (5 ; 105) présente le moyen de fixation, du fait qu'il définit des zones avec des saillies (7) décalées axialement les unes par rapport aux autres, les saillies (7) se présentant, à l'état non expansé, sous la forme de fentes (23) qui, après une compression du premier élément de guidage (5 ; 105) dans la direction axiale, se projettent vers l'extérieur à partir de la surface de l'élément de guidage et sont redressées en formant les saillies finies.

2. Partie du dispositif selon la revendication 1, **caractérisée en ce que** les fentes s'étendent sensiblement parallèlement à l'axe du premier élément de guidage.

3. Partie du dispositif selon la revendication 1 ou 2, **caractérisée en ce que** le premier élément de guidage allongé (5 ; 105) est formé d'un alliage de nickel et de titane ayant un effet de mémoire de forme.

4. Partie du dispositif selon l'une des revendications 1 à 3, **caractérisée en ce que** le premier élément de guidage allongé (5 ; 105) est réalisé sensiblement sous la forme d'un corps creux.

5. Partie du dispositif selon la revendication 4, **caractérisée en ce qu'**une tige adaptée au diamètre intérieur du corps creux est introduite dans le premier élément de guidage allongé (5 ; 105), laquelle présente une courbure prédéterminée qui est adaptée à l'anatomie du lieu d'implantation et transmet la courbure au premier élément de guidage allongé (5 ; 105) et donc au corps tumulaire (3).

6. Partie du dispositif selon l'une des revendications 1 à 5, **caractérisée en ce que** les saillies (7) décalées axialement les unes par rapport aux autres sont décalées d'un angle d'environ 90° les unes par rapport aux autres.

7. Partie du dispositif selon l'une des revendications 1 à 5, **caractérisée en ce que** les saillies (7) décalées axialement les unes par rapport aux autres sont décalées d'un angle d'environ 45° les unes par rapport aux autres.

8. Partie du dispositif selon l'une des revendications 1 à 7, **caractérisée en ce que** les saillies (7) qui se projettent vers l'extérieur à partir de la surface de l'élément de guidage lors de la compression du premier élément de guidage (5 ; 105) dans la direction axiale sont redressées en formant des branches qui forment entre elles un angle α qui est réglable de façon variable.

9. Partie du dispositif selon l'une des revendications 1 à 8, **caractérisée en ce que** le corps tubulaire (3) est réalisé sous la forme d'un tuyau flexible tubulaire en au moins une matière plastique ou un polymère biocompatible, le matériau étant sélectionné parmi les polyuréthanes, les polyéthers bloc amide, les polyamides, le latex, le chlorure de polyvinyle et/ou le silicone.

10. Partie du dispositif selon l'une des revendications 1 à 8, **caractérisée en ce que** le corps tubulaire (3) est réalisé sous la forme d'un tuyau thermorétractable tubulaire en au moins un thermoplastique biocompatible, le matériau étant sélectionné parmi les polyoléfines, le chlorure de polyvinyle, le fluorure de polyvinylidène, le polytétrafluoroéthylène et/ou le Viton, et que ce tuyau et l'élément de retenue de type tuyau s'entremêlent avec la grille métallique (23).

11. Partie du dispositif selon l'une des revendications 1 à 10, **caractérisée en ce que** le corps tubulaire (3) est réalisé sous la forme d'un tuyau flexible tubulaire et pourvu d'un revêtement hydrophile.

12. Partie du dispositif selon l'une des revendications 1 à 11, **caractérisée en ce que** le corps tubulaire (3) est réalisé sous la forme d'un tuyau flexible renforcé par textile et/ou tressé textile, une armature textile simple ou multiple étant présente.

13. Partie du dispositif selon la revendication 12, **caractérisée en ce que** le tuyau thermorétractable tubulaire présente un renfort métallique, et que le renfort métallique ainsi que la grille métallique extensible et compressible de manière réversible de l'élément de retenue de type tuyau sont réalisés sous la forme d'un sandwich entre deux couches de matière plastique.

14. Partie du dispositif selon l'une des revendications 1 à 13, **caractérisée en ce que** le corps tubulaire (3) présente un revêtement en argent antimicrobien ou un revêtement en carbone de type diamant.

15. Partie du dispositif selon l'une des revendications 1 à 14, **caractérisée en ce que** l'élément de retenue de type tuyau est rempli d'air à pression atmosphérique.

16. Partie du dispositif selon l'une des revendications 1 à 15, **caractérisée en ce que** la grille métallique (29) est réalisée sous la forme d'une structure en losanges, les losanges se présentant à l'état comprimé sous la forme de fentes qui présentent de préférence une extension dans la direction longitudinale du tuyau flexible.

17. Partie du dispositif selon l'une des revendications 1 à 16, **caractérisée en ce que** le premier élément de guidage allongé (5 ; 105) débouche à son extrémité distale dans un organe d'actionnement (9) et dépasse de celui-ci pour le réglage manuel ou pour le réglage par l'intermédiaire de la liaison avec un moteur comme entraînement.

18. Partie du dispositif selon l'une des revendications 1 à 17, **caractérisée en ce qu'**elle est conçue réglable activement, et que le premier élément de guidage allongé est réalisé sous la forme d'une crémaillère qui est maintenue dans le corps tubulaire (3) par les saillies (7) et déplaçable par un dispositif de levage mécaniquement à la main ou de façon motorisée.

19. Partie du dispositif selon la revendication 17, **caractérisée en ce que** le dispositif réglable activement est couplé à un capteur de déplacement, d'inclinaison, de pression et/ou de volume et commandé par au moins un de ces capteurs.

20. Dispositif pour empêcher l'incontinence, lequel présente deux parties du dispositif selon l'une des revendications 1 à 19 identiques ou différentes, qui sont implantées chacune au voisinage de l'urètre d'un patient.

21. Dispositif pour empêcher l'incontinence selon la revendication 18, lequel présente au moins une partie du dispositif réglable selon l'une des revendications 1 à 16 et/ou au moins une partie du dispositif selon la revendication 17 et/ou au moins une partie du dispositif réglable activement selon l'une des revendications 18 à 20.
